# EUROPÄISCHE PATENTANMELDUNG

(11) **EP 2 514 372 A1**
(43) Veröffentlichungstag der Anmeldung: **24.10.2012**
(21) Anmeldenummer: 11163566.0
(22) Anmeldetag: 21.04.2011
(51) Int. Cl.: A61B 17/15, A61B 17/17

(54) **Instrumentarium zum Einsetzen einer Gelenkprothese, insbesondere Knieprothese**

(71) Anmelder: Deru GmbH, 22844 Norderstedt (DE)
(72) Erfinder: Amos, Balzarini, 22844 Norderstedt (DE); Iredi, Marco, 22848 Norderstedt (DE); Dmuschewsky, Klaus, 22397 Hamburg (DE)
(74) Vertreter: Glawe, Delfs, Moll

(57) **Zusammenfassung**

Die Erfindung betrifft Instrumentarium zum Einsetzen einer Gelenkprothese, insbesondere einer Femurkomponente einer Knieprothese. Es umfasst einen Basisrahmen (1) mit einer Führplatte (12) und einer seitlich abragende Primärlehre (11), eine Befestigungseinrichtung (99) an dem Knochen, und eine Bogenfräslehre (7) mit einem Grundkörper (70) und einem Führstück (8), das entlang einer gebogenen Führungsbahn (74) gegenüber dem Grundkörper (70) beweglich ist und eine Aufnahme (87) für ein Abtragwerkzeug (85) aufweist. Weiter umfasst es eine Ausrichteinrichtung (17, 77), welche die Bogenfräslehre (7) beim Einsetzen in den Basisrahmen (1) in eine eindeutig definierte Relativposition setzt. Dank der präzisen Positionierung in Verbindung mit der gebogenen Führungsbahn kann eine Präparation des Aufnahmesitzes leicht hergestellt werden, ohne dass es zu einer Beschädigung umliegenden Gewebes kommt. Damit können auch große und kompliziert geformte Prothesen, wie Kniegelenkprothesen, einfach und sicher auf reproduzierbare Weise eingesetzt werden.

## Beschreibung

Die Erfindung betrifft ein Instrumentarium zum Einsetzen einer Gelenkprothese, insbesondere einer Femurkomponente einer Knieprothese, am Ende eines Knochens, insbesondere des Femurs.

Die Implantation einer modernen Gelenkprothese, insbesondere einer Knieprothese, ist eine für den Operateur herausfordernde Tätigkeit. Derartige Gelenkprothesen müssen den komplexen physiologischen Bewegungsverlaufs des natürlichen Gelenks in hohem Maße naturgetreu abbilden. Dies erfordert nicht nur hochentwickelte Prothesen, sondern auch eine präzise Implantation der Gelenkprothese. Nur auf diese Weise kann gewährleistet werden, dass die gewünschte Funktionalität des natürlichen Gelenks korrekt restauriert werden kann. Es versteht sich, dass zur Sicherung eines hinreichenden Therapieerfolges die Positionierung der Prothese nicht nur präzise erfolgen muss, sondern auch sicher und reproduzierbar. Insbesondere darf es nicht zu Beschädigungen oder Verletzungen umgebenden Gewebes, insbesondere umgebenden Knochenmaterials, kommen, da dieses in vielen Fällen für die Tragfunktion eine erhebliche Rolle spielt. Gerade im Hinblick auf eine lange Lebensdauer der Gelenkprothese ergibt sich jedoch ein Zielkonflikt. Zum einen ist es für die lange Lebensdauer günstig, wenn die Kraftübertragung eher großflächig erfolgt. Andererseits kann es jedoch durch eine großflächige und damit zu insgesamt großen Abmessungen der Prothese führende Gestaltung zu einem erhöhten Raumbedarf und damit leicht zu Beschädigungen von umgebendem Gewebe kommen. So besteht die Gefahr, dass in falschen, insbesondere zur Stützung der Gelenkprothese wichtigen Bereichen Material entfernt wird. Es ist daher notwendig, die Implantation auf Grundlage exakter Positionsreferenzen durchzuführen.

Die aus dem einschlägigen Stand der Technik bekannten Instrumentarien ermöglichen zwar eine bewährte Implantation der Gelenkprothese, insbesondere einer Kniegelenkprothese. Jedoch setzen sie eine erhebliche Erfahrung des Operateurs voraus, da das Instrumentarium selbst nur wenig Hilfestellung in Bezug auf eine korrekte Positionierung gibt.

Um schädliche Auswirkungen auf den Patienten durch eine fehlerhafte Positionierung oder durch Wegnahme von zu viel natürlicher Knochensubstanz zu vermeiden, hat sich die vorliegende Erfindung die Aufgabe gestellt, ein verbessertes Instrumentarium zu schaffen, welches eine präzisere Implantation ermöglicht.

Die Aufgabe wird erfindungsgemäß gelöst durch ein Instrumentarium mit den Merkmalen des unabhängigen Anspruchs. Vorteilhafte Weiterbildungen sind Gegenstand der abhängigen Ansprüche.

Bei einem Instrumentarium zum Einsetzen einer Gelenkprothese, insbesondere einer Femurkomponente einer Knieprothese, am Ende eines Knochens, insbesondere des Femurs, ist erfindungsgemäß vorgesehen ein Basisrahmen, der eine Führungsplatte und eine davon seitlich abragende Primärlehre aufweist, eine Befestigungseinrichtung zur positionsfesten Anordnung des Basisrahmens an dem Knochen, eine Bogenfräslehre mit einem Grundkörper und einem Führstück, wobei das Führstück entlang einer gebogenen Führungsbahn gegenüber dem Grundkörper beweglich ist und es eine Aufnahme für ein Abtragwerkzeug aufweist, und eine Ausrichteinrichtung, welche die Bogenfräslehre beim Einsetzen in die Basisplatte in eine eindeutig definierte Relativposition setzt.

Die Erfindung beruht auf dem Gedanken, mittels eines Basisrahmens, der in an sich bekannter Weise in einer Befestigungseinrichtung an dem Knochen positioniert ist, eine stabile und mittels der Ausrichteinrichtung hochgenaue Plattform zu schaffen, an der die Bogenfräslehre leicht einsetzbar ist, und dabei selbsttätig und sicher genau ausgerichtet ist. Die Bogenfräslehre weist eine gebogene Führungsbahn auf, längs der mittels des Abtragwerkzeugs eine entsprechende verrundete Gestaltung des Knochens erfolgen kann. Dies eignet sich insbesondere zur Präparation eines Aufnahmesitzes für Kondylen-Komponenten der Kniegelenkprothese. Deren komplizierte Form kann dank der Erfindung mittels einer Zwangsführung, wie sie durch die gebogene Führungsbahn der Bogenfräslehre erfindungsgemäß erreicht ist, auf einfache und dennoch präzise Weise vom Operateur hergestellt werden. Abweichungen in der Form sind auch bei ungünstigen Operationsverhältnissen dank der Zwangsführung des Abtragwerkzeugs kaum möglich. Damit ergibt sich nicht nur eine hohe Formtreue der somit geschaffenen Präparation, sondern es wird ferner bei korrekter Grundausrichtung des Basisrahmens, der über die Ausrichteinrichtung die Positionierung der Bogenfräslehre eindeutig bestimmt, auch sichergestellt, dass keine Verletzung oder Beschädigung von umliegendem Gewebe erfolgt, insbesondere kein benachbartes Knochenmaterial unnötig abgetragen wird, welches möglicherweise zur Stützung der Prothese benötigt wird. Insbesondere kann so bei Kniegelenkprothesen ein wandartiger Knochenrest am Patellaschild stehen gelassen werden, der nicht nur eine Grundlage für die patellaseitige Abstützung der Gelenkprothese liefert, sondern auch als eine nach frontal wirkende Abgrenzung des kastenförmigen Hohlraums des Markraums zur Aufnahme des Grundkörpers der Prothese dient.

Kurz gesagt ermöglicht die Erfindung auf der Grundlage einer sauberen Grundpositionierung eine exakt definierte Abtragung von Knochenmaterial, wobei auch komplizierte Formen, wie die variierende Krümmung der Kondylen bei einem Kniegelenk, einfach und positionsgenau ausgearbeitet werden können.

Insbesondere zur Anpassung an die, wie bereits vorstehend erwähnt, nicht-konstante Krümmung der Kondylen ist es zweckmäßig, wenn die gebogene Führungsbahn der Bogenfräslehre sich entlang ihrer Erstreckung kontinuierlich in ihrer Krümmung ändert. Mit einer solchen Krümmungsänderung kann der natürliche Bewegungsverlauf des Knies weitgehend funktionsgetreu abgebildet werden. Dabei ist die Geometrie vorzugsweise so gewählt, dass der jeweilige Krümmungsmittelpunkt über die nicht-konstante Krümmung entlang der Führungsbahn hinweg in einer Ebene bleibt, wobei er sich in horizontaler Richtung von anterior nach posterior verschiebt (um vorzugsweise 10 mm, maximal 20 mm). Die Erzeugung eines komplizierten Kurvenverlaufs mit variierender Krümmung, wobei auch die Lage des Mittelpunkts noch bestimmten Anforderungen genügen soll, ist mit den bisherigen Instrumentarien schwierig und wird von diesen kaum oder gar nicht unterstützt. Es kam hierbei im Stand der Technik vielmehr allein oder hauptsächlich auf die Erfahrung und das Geschick des Operateurs an. Mit dem erfindungsgemäßen Instrumentarium wird sichergestellt, dass stets eine präzise Gestaltung des Krümmungsverlaufs gemäß den Vorgaben erreicht werden kann.

Um dies zu erreichen, ist zweckmäßigerweise das Führstück, in welches das Abtragwerkzeug aufgenommen ist, sowohl über einen Folger mit der Führungsbahn wie auch über ein Rotationslager schwenkbar an dem Grundkörper gelagert. Mit dieser doppelten Lagerung wird sowohl die Drehung des Führungsstücks entlang der Führungsbahn gesteuert, wie auch dessen Ausrichtung relativ zur Führungsbahn. Damit kann sowohl der Krümmungsradius wie auch die Position des Mittelpunkts der Krümmung genau gesteuert werden. Besonders bewährt hat es sich, wenn das Rotationslager vom Folger beabstandet angeordnet ist und einen beidseitig schwenkbar gelagerten Kniehebel aufweist. Hierbei ergibt sich eine raumsparende Kinematik, die darüber hinaus präzise Führung verknüpft mit einer Vermeidung von Verklemmungen. Gerade letzteres ist ein bedeutender Aspekt, da es im Operationsumfeld wegen der allgegenwärtigen Gefahr von Fremdkörpereintritt (insbesondere Gewebereste oder Körperflüssigkeiten) leicht zu einem Blockieren gerade bei hoch präzise geführten technischen Einrichtungen kommen kann. Die Kombination aus einem Folger mit einem beidseitig schwenkbar gelagerten Kniehebel ist in dieser Hinsicht robust.

Besonders bevorzugt ist es, wenn der Kniehebel über eine Sicherung abnehmbar an dem Grundkörper gehaltert ist. Dies ermöglicht es, die Bogenfräslehre mit ihrem Grundkörper gesondert in die Basisplatte einzusetzen, ohne dabei durch das über einen großen Verstellbereich bewegliche Führstück behindert zu werden. Dies stellt nicht nur eine Vereinfachung dar, sondern ermöglicht eine bessere Handhabung. Dies gilt gerade auch für den Fall, wenn wegen geringer Toleranzen das Einsetzen der Bogenfräslehre schwierig ist, wobei die dazu erforderliche Kraft gegebenenfalls mit einem Hammer aufgebracht werden kann. Dank der Abnehmbarkeit des Führstücks besteht dann bei dem Eintreiben des Grundkörpers keine Gefahr, das für die genaue Positionierung entscheidende Führstück mit seiner Führungsbahn zu beschädigen.

Um im Operationsumfeld eine umständliche Montage des Führstücks zu vermeiden und auch der Gefahr eines Verlierens von einzelnen Montageteilen vorzubeugen, ist zweckmäßigerweise eine Schnellkupplung zwischen Führstück und Grundkörper vorgesehen. Besonders bewährt hat sich eine Ausführung als Winkelverriegelung, die durch Verbringen des Führstücks in einen Fortsatz der Führungsbahn trennbar ist. Unter einem Fortsatz wird hierbei ein Bereich der Führungsbahn verstanden, der für die eigentliche Formgebung mittels des Abtragwerkzeugs nicht erforderlich ist (sondern sozusagen einen nicht genutzten Zusatzbereich darstellt). Indem das Führstück in diesen Fortsatz gebracht wird, gelangt die Winkelverriegelung in eine solche Stellung, dass sie leicht und werkzeuglos getrennt werden kann. Entsprechendes gilt für die Montage, die in gleicher Weise leicht und werkzeuglos erfolgen kann. Dazu braucht das Führstück lediglich in die Extremposition in den Fortsatz gebracht zu werden, um dann getrennt oder wieder aufgesetzt werden. Im Bereich des Fortsatzes ist dies zweckmäßigerweise dadurch kenntlich gemacht, dass dort eine Öffnung der Führungsbahn nach draußen besteht. Unbedingt erforderlich ist diese Öffnung zwar nicht, jedoch erleichtert sie ein Entnehmen des Führstücks in dieser Position. Es versteht sich, dass diese Öffnung im Übrigen, nicht zu dem Fortsatz gehörenden Bereich der Führungsbahn entsprechend nicht vorhanden ist.

Die Winkelverriegelung kann bestehen aus einer Drehlagerhülse und einem nicht-kreisförmigen Drehzapfen, der so ausgebildet ist, dass die Winkelverriegelung nur in einer Winkelposition des Führstücks öffnet. Dies wird in der Weise erreicht, indem die Drehlagerhülse über eine Verengung zu einer Seite hin offen ist. Der nicht-kreisförmige Drehstift ist so beschaffen, dass er je nach Positionierung unterschiedliche Breiten aufweist. Diese Breite in einer bestimmten Positionierung wird als die Breite in einem bestimmten Meridian bezeichnet. Weist der nicht-kreisförmige Drehstift also eine solche Form auf, die im Wesentlichen einem Rechteck mit kurzen kreisbogenförmigen Seiten entspricht, so ist die Breite in dem Meridian kleinster Weite gleich dem Abstand der beiden langen Seiten des Rechtecks, und die Breite entsprechend dem Meridian größter Weite gleich der Länge der längeren Seite des Rechtecks zuzüglich den Hervorwölbungen durch die kreisbogenförmigen kurzen Seiten. Indem die Verengung so gewählt ist, dass sie zwar für den Durchgang des Drehzapfens in seinem Meridian kleinster Breite, nicht aber für den Durchgang in einem Meridian größter Breite ausreicht, kann das Führstück mit seinem Drehzapfen lediglich in einer solchen Orientierung abgenommen werden, in der der Drehzapfen mit seinem Meridian kleinster Breite die Verengung passieren kann. Dies ist erfindungsgemäß nur dann der Fall, wenn sich das Führstück in der Position der Führungsbahne befindet, die zu dem Fortsatz gehört. In den übrigen Positionen an der Führungsbahn ist das Führstück verriegelt, da dort die Breite des Drehzapfens größer ist als die Weite der Verengung, so dass der Drehzapfen nicht durch die Verengung entnommen werden kann. Damit ergibt sich eine einfache und robuste Schnellkupplung, die winkelgesteuert ist und damit die Gewähr bietet, dass die Trennung nur in einer bestimmten Position erfolgen kann. Die Montage des Führstücks gelingt ebenfalls leicht, denn es braucht nur in der richtigen Position in den Fortsatz der Führungsbahn eingeschoben zu werden, wobei der Drehzapfen mit seinem Meridian geringster Breite ohne weiteres durch die Verengung in die Drehlagerhülse geführt wird. Damit wird eine einfache, werkzeuglose Verriegelung durch ein Hineinschieben des Führstücks in seiner Position erreicht.

Der Kniehebel ist vorzugsweise ebenfalls abnehmbar an den Grundkörper gehaltert. Damit ist es ermöglicht, sämtliche beweglichen Teile abzunehmen. Dies ist nicht nur günstig für die Reinigung des Instrumentariums, sondern auch, wie bereits ausgeführt, für die Montage des Grundkörpers in schwierigen Fällen, ohne dass es zu einer Beschädigung der empfindlichen Führeinrichtungen kommt. Vorzugsweise erfolgt die Halterung des Kniehebels über eine Sicherung, so dass er gegen ein unbeabsichtigtes Lösen aus der vorgesehenen Position geschützt ist. Als Sicherung hat sich insbesondere eine Schraube bewährt.

Das entlang der Führungsbahn bewegliche Führstück definiert durch seine Aufnahme eine Achse für das abtragende Werkzeug. Die Achse kann so orientiert sein, dass sie in der Schwenkebene des Führstücks liegt, vorzugsweise ist sie jedoch schiefwinklig. Weiter vorzugsweise ist diese Achse ebenfalls schiefwinklig zur Linie zwischen Folger und Rotationslager. Bewährt hat sich hierfür ein Bereich zwischen 10° und 35°, weiter vorzugsweise zwischen 15° und 30°. Damit wird eine Knickreserve an dem Kniehebel geschaffen, die eine auch weitergehende Veränderung des Radius entlang des Verlaufs der Führungsbahn zulässt.

Vorzugsweise wirkt die Aufnahme für das Abtragwerkzeug an dem Führstück mit einem Tiefenanschlag zusammen. Unter Tiefenanschlag wird hierbei eine Einrichtung verstanden, welche die Eindringtiefe des Abtragwerkzeugs in das Werkstück, hier also den Knochen an dem das erfindungsgemäße Instrumentarium verwendet wird, begrenzt. Bewährt hat sich eine Ausführung des Tiefenanschlags in der Weise, dass es ein gestufter Sitz ist, der vorzugsweise zu einer Seite hin offen ist. Mit der Stufung wird erreicht, dass eine entsprechend an den tiefen Fräser angeordnete Verdickung dort aufliegt und damit einen Anschlag in Bezug auf die Eindringtiefe des Tragwerkzeugs bildet. Durch die seitliche Öffnung ist sichergestellt, dass das Abtragwerk direkt von der Seite in die Aufnahme eingesetzt werden kann, ohne dass es umständlich eingefädelt zu werden braucht.

Grundsätzlich kann es zwar ausreichen, dass nur eine Aufnahme an dem Führungsstück vorgesehen ist, jedoch ist es gerade für Knieprothesen zur Ausbildung von zwei Kondylen von Vorteil, wenn am Führstück eine Doppelaufnahme ausgebildet ist. Damit kann das Abtragwerkzeug umgesetzt werden, nachdem eine Kondyle ausgebildet ist, um so an der anderen Position die zweite Kondyle auszubilden. Eine Demontage bzw. ein Umsetzen der Bogenfräslehre ist dazu nicht erforderlich, so dass die präzise Positionierung erhalten bleibt.

Es hat sich bewährt, wenn die Doppelaufnahme so an dem Führstück ausgebildet ist, dass sich divergierende Achsen ergeben. Unter divergierend wird hierbei verstanden, dass das Abtragwerkzeug mit seinem Schneidkopf im eingesetzten Zustand nach außen weist. Damit kann den Kondylen eine physiologische Neigung gegeben werden, die eine beim natürlichen Kniegelenk vorhandene Selbstzentrierungsfunktion unterstützt.

Zwingend erforderlich ist die Doppelaufnahme jedoch nicht. Es kann auch vorgesehen sein, dass das Führstück mit dem Rotationslager und gegebenenfalls mit dem Kniehebel von einer Seite des Grundkörpers auf die andere ummontierbar ist. Damit kann zuerst auf einer Seite die Kondyle präpariert werden, und nach Ummontieren auf der anderen Seite mit derselben Aufnahme am Führstück die andere Kondyle präpariert werden. Da auch hierbei die Positionierung der Bogenfräslehre mit ihrem Grundkörper an sich nicht verändert zu werden braucht, bleibt die präzise Positionierung erhalten.

An dem Grundkörper und/oder der Führungsbahn sind zweckmäßigerweise Befestigungsbohrungen vorgesehen. Sie ermöglichen eine Sicherung der Positionierung der Bogenfräslehre, und zwar unabhängig von ihrer Aufnahme in die Basisplatte. Damit werden die Befestigungssicherheit und damit letztlich auch die Güte der Positionsgenauigkeit erhöht; insbesondere ist es ermöglicht, den Basisrahmen abzunehmen.

Gemäß einem besonderen Aspekt der Erfindung, der gegebenenfalls unabhängigen Schutz verdient, sind verschiedene Einsätze für den Basisrahmen vorgesehen. So kann ein Ausricht-Einsatz an dem Basisrahmen wechselbar vorgesehen sein, welcher zur Aufnahme eines Ausrichtkörpers in definierter Position ausgebildet ist. Bei dem Ausrichtkörper kann es sich insbesondere um ein Knochenräumwerkzeug, insbesondere eine Ahle bzw. eine Raspel zur Öffnung des Markraums handeln. Das in vorangegangenen Absätzen beschriebene Führstück wird meistens so ausgeführt sein, dass die Aufnahme für das Abtragwerkzeug mit seiner Achse so orientiert ist, dass die Achse eine radiale Orientierung aufweist. Das bedeutet, dass sie im Wesentlichen zum Krümmungsmittelpunkt hinaus gerichtet ist. Es kann aber alternativ auch vorgesehen sein, dass die Achse quer zur durch die krümmungsdefinierte Ebene orientiert ist. Dies bietet den Vorteil, dass das Abtragwerkzeug von der Seite aus in die Aufnahme einsetzbar ist. Bei einem hinreichend großen Abtragwerkzeug können damit beide Kondylenformen an einem Femur mit einer Bewegung entlang der Führungsbahn geformt werden. Vorzuziehen ist aber eine Variante, bei der das Führstück mit dem Rotationslager und gegebenenfalls den Kniehebel von einer Seite des Grundkörpers auf die andere ummontierbar ist. In diesem Fall ist das Abtragwerkzeug so dimensioniert, dass nur eine Kondylenbahn, nämlich die jeweils nächstliegende geformt ist. Dies bietet den Vorteil einer präziseren Kontrolle und ermöglicht es ferner, durch eine gegebenenfalls leicht verkippte Achse in der Aufnahme des Führstücks der Kondylenbahn eine Neigung zu geben. Damit wird ein vergleichbares Ergebnis erzielt, wie es mit den divergierenden Achsen bei dem oben beschriebenen Führstück mit einer Doppelaufnahme erreichbar ist.

Um eine unabhängige Befestigung der Bogenfräslehre am zu bearbeitenden Knochen zu erreichen, können zweckmäßigerweise Befestigungsbohrungen am Grundkörper und/oder der Führungsbahn vorgesehen sein. Sie dienen in erster Linie als Ersatz zu der Befestigung mittels der Ausrichteinrichtung an dem im Knochen verankerten Basisrahmen, so dass der Basisrahmen bei Bedarf abgenommen werden kann.

Gemäß einem weiteren Aspekt der Erfindung, der gegebenenfalls unabhängigen Schutz verdient, sind für den Basisrahmen ein Ensemble von Einsätzen vorgesehen, welche wechselbar in die Führplatte aufnehmbar sind. Hierbei handelt es sich insbesondere um einen Ausricht-Einsatz, einen Frontalsäge-Einsatz, einen ersten Fräs-Einsatz, einen zweiten Fräs-Einsatz und einen dritten Fräs-Einsatz mit einer Kulissenführung.

Der Ausricht-Einsatz ist dazu ausgebildet, in definierter Position an der Führplatte wechselbar angeordnet zu sein, wobei er eine Aufnahme für den Ausrichtkörper aufweist. Damit kann die Basisplatte eindeutig in Bezug auf den Ausrichtkörper positioniert sein. Zweckmäßigerweise ist der Ausricht-Einsatz seitenabhängig, das heißt es gibt einen Ausricht-Einsatz "L" für eine Implantation der linksseitigen Prothese und einen Ausricht-Einsatz "R" für die Implantation einer rechtsseitigen Prothese. Es sei angemerkt, dass als Ergänzungen zusätzlich auch ein symmetrischer und damit beidseitig in gleicher Weise benutzbarer Hilfs-Ausrichteinsatz vorgesehen sein kann.

Die Aufnahme für den Ausrichtkörper an dem Ausricht-Einsatz ist zweckmäßigerweise einseitig offen. Bevorzugt ist dies mittels einer Verengung bewerkstelligt. Damit wird erreicht, dass der Ausrichtkörper (in der Regel wird es sich hier um ein in den Markraum des Knochens eingeschobenes Instrument, wie eine Ahle oder einen Fräser, handeln) leicht von der Seite her in die Aufnahme eingesetzt bzw. aus dieser entnommen werden kann.

Weiter umfasst das Ensemble an Einsätzen einen FrontalSäge-Einsatz. Dieser weist zwei V-förmig zueinander ausgerichtete Sägeschlitze und eine bipolare Befestigung auf. Unter der bipolaren Befestigung wird eine Befestigung verstanden, welche zwei alternative Befestigungspositionen definiert. Diese Befestigungspositionen sind so gewählt, dass die Sägeschlitze einmal zur Implantation einer linksseitigen Prothese und im anderen Fall zur Implantation einer rechtsseitigen Prothese angeordnet sind. Eine zweckmäßige Ausführungsform für eine solche bipolare Befestigung können zwei einzelne Befestigungsbohrungen sein, oder vorzugsweise ein Langloch, dessen Endpunkte die jeweiligen bipolaren Befestigungspositionen definieren.

Vorzugsweise umfasst das Instrumentarium weiter Abstandshalter für verschiedene Höhen, die zur beidseitigen Anordnung am Rand der Führplatte ausgebildet sind. Mit den Abstandshaltern kann ein bestimmter Abstand der Führplatte zu dem Knochen eingestellt werden. Dies eignet sich insbesondere für solche Fälle, bei denen bereits aufgrund einer vorhergehenden Operation Knochenmaterial entfernt worden ist. Auf diese Weise kann der Materialverlust ausgeglichen werden. Vorzugsweise sind Abstandshalter mit verschiedenen Abmessungen im Instrumentarium enthalten.

Weiter umfasst das Ensemble einen ersten Fräs-Einsatz, der wechselbar in der Führplatte anbringbar ist. Er bildet eine definierte Aufnahme für einen Räumfräser, die vorzugsweise auch einen Tiefenanschlag für den Räumfräser bildet. Damit ist eine genaue Durchführung der Fräsung sichergestellt. Insbesondere wird damit erreicht, dass der Räumfräser nicht zur Seite auswandert und damit lateral oder frontal bzw. dorsal in unerwünschter Weise Knochenmaterial abträgt. Mit dem Tiefenanschlag ist weiter sichergestellt, dass nur in der für die Implantation erforderlichen Tiefe Knochenmaterial abgetragen wird. Der Abstand der Aufnahme zur Primärlehre ist so bemessen, dass bei eingesetztem Räum-Fräser ein Abstand verbleibt, welcher der Dicke einer an der Frontalseite zum Patellaschild zu belassenden Wandung entspricht. Dies gelingt dank der damit erreichten Zwangsführung des Räumfräsers auch bei einem unübersichtlichen Operationsumfeld, und zwar auch einem weniger erfahrenen Chirurgen.

Vorzugsweise umfasst das Instrumentarium weiter eine Fühlerlehre, die an einer Steckaufnahme an dem Basisrahmen winkelgenau anbringbar ist. Mittels dieser Fühlerlehre kann die Lage des Drehpunkts der Prothese angezeigt werden, und zwar vorzugsweise in zwei Orientierungsebenen.

Weiter umfasst das Ensemble einen zweiten Fräs-Einsatz, der wechselbar an der Führplatte anbringbar ist. Dieser weist eine Doppelaufnahme zur umsteckbaren Aufnahme eines Volumenfräser auf. Hierbei ist die Doppelaufnahme vorzugsweise so ausgebildet, dass sie verschiedene Tiefenanschläge und außerdem einen Lateralversatz aufweist. Unter Lateralversatz wird hierbei verstanden, dass der Volumenfräser in der einen Position der Doppelaufnahme in Lateral-Medial-Richtung anders positioniert ist als in der anderen Position der Doppelaufnahme. Gleiches gilt in Bezug auf den Tiefenanschlag, nämlich dass der Volumenfräser in einer der beiden Positionen eine größere Frästiefe erreicht als in der anderen Position. Die Doppelaufnahme ist vorzugsweise so ausgebildet, dass sich ihre Bereiche überlappen. Damit wird erreicht, dass nach der Fräsung ein verbundener Hohlraum im Knochen entsteht. Dieser bildet die Grundlage zur weiteren Ausarbeitung, um so eine definierte Form zur präzisen Implantation herstellen zu können. Damit kann ein maximal großer Raum ausgefräst werden, ohne dass dabei die Gefahr besteht, die umgebende Knochenwandung zu verletzen. Es sei angemerkt, dass als zweiter Fräs-Einsatz auch ein Hilfs-Fräs-Einsatz vorgesehen sein kann, der ebenfalls eine Doppelaufnahme aufweist. Diese Doppelaufnahme ist jedoch vereinfacht in dem Sinne, dass sie vorzugsweise identische tiefe Anschläge hat und/oder keinen Lateralversatz aufweist. Damit können einfachere Strukturen für den Hohlraum hergestellt werden. Dieser Hilfs-Fräs-Einsatz ist insbesondere dann zweckmäßig, wenn nur en verhältnismäßig kleiner Hohlraum ausgearbeitet zu werden braucht.

Das Ensemble umfasst weiter einen dritten Fräs-Einsatz, der an der Führplatte anbringbar ist. Dieser bildet eine Kulissenführung für einen Tiefenfräser, der vorzugsweise in einen Kulissengleiter aufgenommen ist. Mit der Kulissenführung kann eine Feinausformung des Hohlraums mittels des Tiefenfräsers erreicht werden. Die Kulisse begrenzt hierbei die Bewegung des Tiefenfräsers in Lateral-Medial-Richtung. Weiter kann vorgesehen sein, dass mittels eines Kulissenfensters die Bewegung des Fräsers in Frontal-Dorsal-Richtung begrenzt ist. Vorzugsweise weist der Kulissengleiter einen Handgriff auf, der den Tiefenfräser koaxial umgibt. Damit kann eine präzisere Führung des Tiefenfräsers erreicht werden. Die Kulissenführung kann weitere Fenster zur visuellen Kontrolle der Fräsung aufweisen.

Vorzugsweise weist die Kulissenführung einen Tiefenanschlag auf. Somit kann auch ein zweiter Tiefenfräser vorgesehen sein, so dass durch zwei verschiedene Frästiefen definiert sind. Dies ermöglicht eine sichere und präzisere Ausarbeitung auch komplexer Hohlräume.

Bei einer alternativen Ausführungsform kann auch vorgesehen sein, dass die Kulissenführung zwei gelenkig miteinander verbundene Führhebel umfasst. Hierbei kann an einem Ende die Aufnahme für den Tiefenfräser angeordnet sein, während am anderen Ende der gelenkig miteinander verbundenen Führhebel eine schwenkbare Lagerung am Einsatz in die Führplatte vorgesehen ist. Somit ergibt sich eine genauere Führung des Fräsers im Sinne einer Zwangsführung. Weiterhin kann diese Ausführung einen Vorteil haben, dass eine Verkantungsgefahr verringert ist. Zweckmäßigerweise ist die Lagerung am Einsatz so gestaltet, dass die gelenkig verbundenen Führhebel nur aufsetzbar bzw. trennbar sind, wenn das Fräswerkinstrument nicht eingesetzt ist.

Vorzugsweise ist an der Führungsplatte eine Einschubführung vorgesehen, die insbesondere als eine Schwalbenschwanzführung ausgebildet ist. Damit können die verschiedenen Einsätze des Ensembles, wie sie vorstehend beschrieben sind, auf einfache Weise eingesetzt und präzise in Bezug auf die Führplatte positioniert werden.

Das Instrumentarium umfasst mit Vorteil weiter eine Setzzange für die Bogenfräslehre. Diese ergreift die Bogenfräslehre formschlüssig in einer definierten Position, wobei die Setzzange über Ausrichtnasen mit der Ausrichteinrichtung so zusammenwirkt, dass die Setzzange eine eindeutige Position und damit auch die von der Setzzange formschlüssig in eindeutiger Position gehaltene Bogenfräslehre eindeutig relativ zur Führplatte positioniert ist. Damit ergibt sich eine beträchtliche Handhabungsvereinfachung, da die in der Regel verhältnismäßig große Abmessungen aufweisende Bogenfräslehre so sicher und genau positioniert werden kann. Eine Fehlpositionierung ist damit ausgeschlossen.

Das Instrumentarium umfasst zweckmäßigerweise ferne Ausrichtstangen zur seitlichen Anordnung am Basisrahmens derart, dass sie voneinander weg weisen. Zur Montage dieser Ausrichtstangen sind Ausrichtbohrungen an Lateralseiten des Basisrahmens vorgesehen.

Weiter umfasst das Instrumentarium einen Bohrer mit einem Tiefenanschlag. Er ist dazu ausgebildet, auf positionsgenaue und einfache Weise am Knochen Aufnahmen für Verankerunszapfen der Endoprothese zu schaffen.

Das Instrumentarium umfasst ferner verschiedene Ahlen zum Ausräumen einer Höhlung am Knochen, insbesondere eines Markraums am Femur. Weiter umfasst es zweckmäßigerweise eine Raspel-Räumahle, deren Schaft einen Einstich aufweist. Dieser ist zur Aufnahme eines Anschlagtellers ausgebildet, der insbesondere als Tiefenanschlag fungiert. Die Raspel-Räumahle ist zweckmäßig so gestaltet, dass sie 2, 3 oder 4 Schneidkanten aufweist. Hierbei sind die Zähne an den Schneidkanten mit relativem Höhenversatz zueinander angeordnet. Dadurch wird bewirkt, dass bei Drehung die Zähne an verschiedenen Stellen Knochenmaterial abtragen, so dass sich eine Glättung der Knochenoberfläche ergibt.

Das Instrumentarium umfasst zweckmäßigerweise weiter eine Richtlehre, welche zur Positionierung der Basisplatte vorgesehen ist und die an der Ausrichteinrichtung angreift. Insbesondere ist sie dazu ausgebildet, an der im Markraum steckenden Raspel-Räumahle anzugreifen, und damit die Basisplatte exakt in Bezug auf diese zu positionieren.

Die Erfindung wird nachfolgend in Bezug auf die beigefügten Zeichnungen näher erläutert, indem ein vorteilhaftes Ausführungsbeispiel dargestellt ist. Es zeigen:
- Fig. 1: eine Darstellung eines Basisrahmens;
- Fig. 2: eine Reibahle;
- Fig. 3: Elemente zum Ausrichten des Basisrahmens;
- Fig. 4: Säge- und Fräslehren zum Vorbereiten eines Hohlraums am Ende eines Femurs;
- Fig. 5: eine Kulissenfräslehre zur Feinbearbeitung des Hohlraums;
- Fig. 6: Werkzeuge zur Vorbereitung eines Markraums;
- Fig. 7: eine Bogenfräslehre umfassend einen Grundkörper;
- Fig. 8: ein Führstück für die Bogenfräslehre gemäß Figur 7;
- Fig. 9: einen Kondylenfräser eingesetzt in das Führstück gemäß Figur 8;
- Fig. 10: eine Einsetzzange für die Bogenfräslehre in die Basisplatte;
- Fig. 11: Einzeldarstellungen zur Anordnung des Führstücks am Grundkörper der Bogenfräslehre;
- Fig. 12: eine Darstellung der Kinematik des Führstücks an dem Grundkörper;
- Fig. 13: alternative Ausführungen des Führstücks;
- Fig. 14: Einzelheiten zur Ausrichtung des Basisrahmens;
- Fig. 15: Einzelheiten zur Verwendung von Ausgleichsstücken gemäß Figur 3;
- Fig. 16: Einzelheiten zur Anwendung des Fräsers gemäß Figur 3;
- Fig. 17: eine alternative Befestigung von den Abstandshaltern;
- Fig. 18: Einzelheiten zur Verwendung der Sägelehre gemäß Figur 4;
- Fig. 19: eine alternative Ausführung der Richtlehre;
- Fig. 20: eine Frontalansicht zur Verwendung der ersten Fräslehre mit Fräser gemäß Figur 4;
- Fig. 21: eine Alternative zur Verwendung des ersten Fräsers;
- Fig. 22: Einzelheiten zur Anwendung der zweiten Fräslehre gemäß Figur 4;
- Fig. 23: Einzelheiten zur Anwendung der Kulissen-Fräslehre gemäß Figur 5;
- Fig. 24: eine alternative Kulissen-Fräslehre;
- Fig. 25: Sicherungen für Einsätze; und
- Fig. 26: Darstellungen zum Zusammenwirken von Sicherungen und Einsätzen.

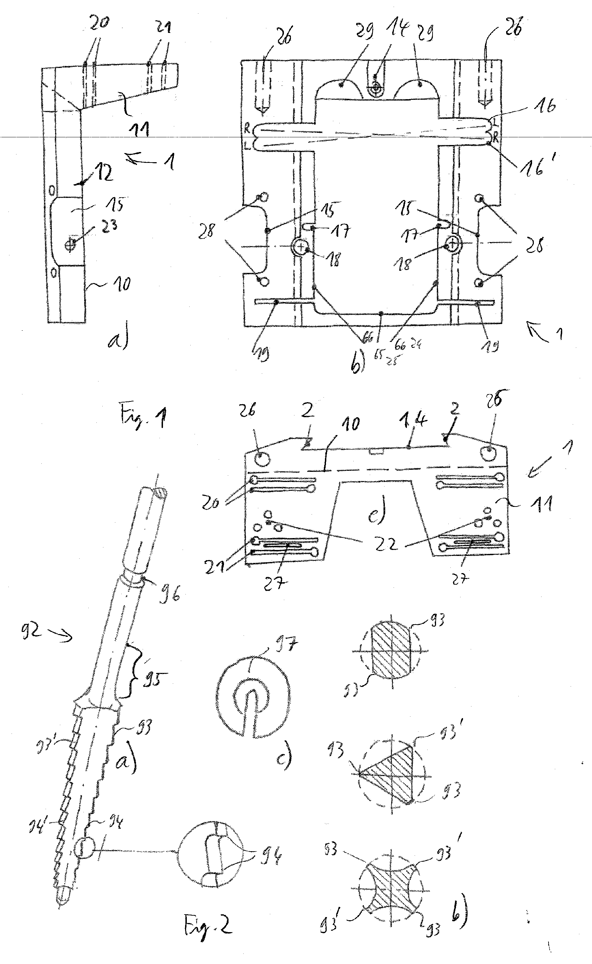

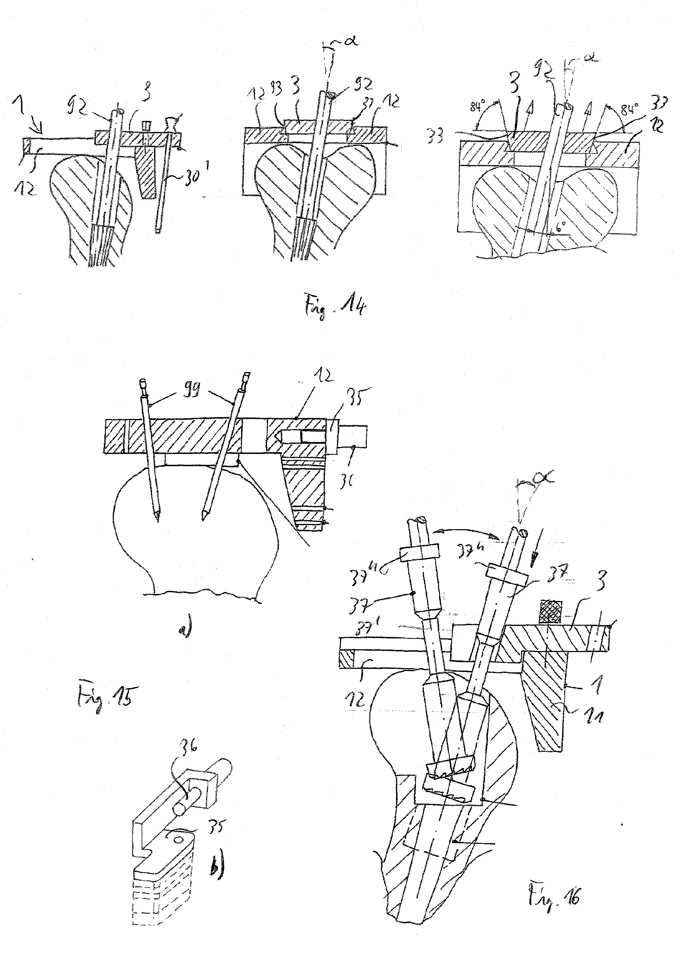

Das als Ausführungsbeispiel in den Figuren 1-11 dargestellte Instrumentarium zur Implantation einer Kniegelenkendoprothese umfasst zwei Ahlen 90, 91 (s. Fig 14) sowie eine Raspel-Räumahle 92 (s. Fig. 2), einen Basisrahmen 1, Stifte 99 als Befestigungseinrichtung an einem Femurknochen, einen Ausricht-Einsatz 3, einen Frontalsäge-Einsatz 40, Abstandshalter 34, eine Fühlerlehre 39, einen ersten Fräs-Einsatz 4, einen zweiten Fräs-Einsatz 5, einen dritten Fräs-Einsatz 6 als Kulissenführung, und eine Bogenfräslehre 7 mit einem Kondylenfräser 79.

Der Basisrahmen 1 umfasst eine Führungsplatte 12 und eine davon orthogonal seitlich abragende Primärlehre 11. An der Primärlehre 11 sind im oberen Bereich eine erste Sägeblattführung 20, die zum Ausgleich unterschiedlicher Kondylenhöhen dient, und im unteren Bereich eine zweite Sägeblattführung 21 ausgebildet, die zur Bearbeitung der Kondylen bei Verwendung femoraler Segmente dient. Weiter sind im Bereich zwischen den Sägeblattführungen Bohrungen 22 für Befestigungspins ausgebildet. Im Übergangsbereich zur Führungsplatte 12 sind in Richtung der Erstreckung der Führungsplatte 12 ausgerichtete Aufnahmebohrungen 26 für Abstandshalter vorgesehen.

Der Basisrahmen 1 ist in verschiedenen Größen vorgesehen. Hierbei ist der Abstand zwischen der Bezugsebene 10 und den Sägeblattführungen 20, 21 sowie zu den Aufnahmebohrungen 26 zu den Abstandshaltern identisch für die verschiedenen Größen.

Die Führungsplatte 12 ist mit ihrer Unterseite als Bezugsebene 10 ausgeführt. Diese dient als Anlagefläche zu Kondylen des Femurs. An der Oberseite ist eine Planizität ausgearbeitet, die Werkzeugebene 14 fungiert. Sie ist die Bezugsebene für die verschiedenen Einsätze, die an der Führungsplatte 12 aufgenommen werden. Hierbei ist das Maß zwischen der Bezugsebene 10 und der Werkzeugebene 14 auf die jeweilige Größe des einzusetzenden Implantats abgestimmt. Beidseitig der Werkzeugebene 14 ist eine Linearführung 2 angeordnet, die zwei hinterschnittene Kanten zwischen der Werkzeugebene 14 und der Oberseite der Führungsplatte 12 umfasst. Sie wirken als Schwalbenschwanzführung für die Einsätze 3, 4, 5 und 6.

An der Führungsplatte 12 ist an den Lateralseiten jeweils eine Einfräsung 15 ausgebildet. Sie ist so tief ausgeführt, dass die zwischen ihnen verbleibende Weite der Breite des zu der jeweiligen Basisplatte 1 zugeordneten Breitenmaßes des Implantats entspricht. Die Einfräsung 15 fungiert somit als Sichthilfe zur Auswahl der Implantatgrößen. In der Einfräsung 15 ist je eine Ausrichtbohrung 23 mit einem Innengewinde vorgesehen, welche mittels einer einzuschraubenden Ausrichtstange 38 die Lage anzeigt. Flankierend zu den Einfräsungen 15 sind Bohrungen 28 für Befestigungsstifte 99 vorgesehen. Im Mittelbereich der Führungsplatte 12 ist eine zentrale Öffnung einer etwa rechteckförmigen Grundform ausgebildet, die an ihren beiden zur Seite weisenden Lateralflächen als seitliche Begrenzung 66 und mit ihrer von der Primärlehre abgewandten Kante 65 als hintere Begrenzung für eine Kastenfräsung fungiert. In den Lateralseiten 66 sind Führungsschlitze 17 für ein Einsetzinstrument 76 der Bogenfräslehre 7 ausgebildet. Dazu benachbart sind in der Führungsplatte 12 Bohrerführungen 18 für eine Stiftlochbohrung beidseitig vorgesehen. In dem rückwärtigen Bereich nahe der hinteren Begrenzung 65 für die Kastenfräsung ist eine posteriore Sägeblattführung 19 zur Kondylenbearbeitung ausgebildet. An dem gegenüberliegenden Ende der großen Zentralöffnung ist in dem zur Primärlehre 11 weisenden Ende eine parallel zu dieser orientierte diagonale Wechselführung 16, 16' ausgebildet. Sie weist an ihren jeweiligen Seitenflächen eine doppelte Bogenform auf und bildet damit eine bipolare Aufnahme für den Frontalsägeeinsatz 40. Dieser ist zur Verarbeitung für eine linksseitige Position in der mit der Bezugsziffer 16 bezeichneten Position und zur Bearbeitung einer rechtsseitigen Implantation in der mit der Bezugsziffer 16' bezeichneten Positionierung eingesetzt.

Oberhalb der Primärlehre 11 ist an der Basisplatte 12 mittig eine Zentralbefestigung 14 vorgesehen. Sie dient dazu, die einzelnen Einsätze zu befestigen. Sie befindet sich für die verschiedenen Größen am Basisrahmen 1 immer an derselben Stelle, so dass die verschiedenen Einsätze problemlos bei Basisrahmen 1 unterschiedlicher Größe verwendet werden können. Beidseitig davon sind Freiarbeitungen 29 ausgebildet, die für Befestigungspins an der Bogenfräslehre 7 zu deren Befestigung am Femur den erforderlichen Freiraum schaffen.

Die in ihrer Gesamtheit mit der Bezugsziffer 92 bezeichnete Raspel-Räumahle umfasst eine Mehrzahl an Schneidkanten 93 in ihrem unteren Bereich, die jeweils mit einer Vielzahl von Zähnen 94 versehen sind. An die mit den Zähnen 94 versehenen Schneidkanten 93 schließt sich nach oben ein zahnfreier Bereich 95 mit verringertem Durchmesser an. Oberhalb davon ist ein Einstich 96 ausgebildet. Dieser dient als Aufnahme für einen Anschlagteller 97. Die Schneidkanten 93 sind vorzugsweise in einer Dreieckkonfiguration ausgeführt, das bedeutet es sind drei Schneidkanten 93 vorgesehen, die in einem Winkelabstand von 120° angeordnet sind. Es sei angemerkt, dass auch eine andere Anzahl von Schneidkanten, insbesondere zwei Schneidkanten bzw. vier Schneidkanten vorgesehen sein könnten (s. Darstellungen in Fig. 2b). Bei der in Fig. 2a dargestellten Ausführungsform ist die Anordnung der Zähne 94 so gewählt, dass die Zähne 94 einer Schneidkante 93 in der Höhe gemessen von der Spitze der Raspel-Räumahle 92 versetzt zu Zähnen 94' der benachbarten Schneidkante 93' angeordnet sind. Dies hat den Vorteil, dass bei der Drehung der Raspel-Räumahle 92 eine gleichmäßigere Gestaltung der Knochenwandung erreicht wird. Sie ist speziell dazu ausgebildet, den Knochen im randnahen Bereich zu erhalten. Dazu weist sie eine Abflachung auf, mit der sie zum Knochenrank orientiert ist. Nach dem Einbringen in die Tiefe der Markraumhöhle und dem Raspeln wird sie dann als Reibahle benutzt.

Die Raspel-Räumahle 92 fungiert mit ihrem oberen Schaftbereich oberhalb des Einstichs 96 als Ausrichthilfe und wirkt dazu zusammen mit dem Ausrichteinsatz 3. Dieser weist eine Öffnung 31 auf, die über eine Verengung 31' zur Seite hin offen ist. Darin ist die Raspel-Räumahle 92 mit ihrem Einstich 96 in die Öffnung 31 einbringbar, indem sie durch die Verengung 31' eingesetzt ist. Dadurch wird eine relative Positionierung zwischen der Raspel-Räumahle 92 und der Basisplatte 1 erreicht, in welcher der Ausricht-Einsatz 3 eingesetzt ist. Die Ausrichtstangen 38 werden in die Ausrichtbohrungen 23 eingeschraubt und zeigen dabei dem Operateur die Lage des Basisrahmens an und fungieren so als Ausrichthilfe. Im dargestellten Ausführungsbeispiel ist die Öffnung 31 nicht orthogonal an Bezugsebene 10 orientiert, sondern steht schiefwinklig zu ihr. Die Abweichung von der orthogonalen Richtung ist als Schaftwinkel α bezeichnet und ein kennnzeichnendes Maß für die Prothese (im Beispiel 6 Grad). Um dem Operateur diesen Schaftwinkel zu visualisieren, ist an dem Ausricht-Einsatz 3 weiter eine Öffnung 30 ausgebildet, die an einem über die vordere Begrenzung der Basisplatte 1 herausragende Zunge ausgebildet ist, durch diese Öffnung 30 ist ein Taststift 30' gesteckt, welcher außerhalb des zu bearbeitenden Femurs liegt und dem Operateur damit den Schaftwinkel der im Markraum des zu bearbeitenden Femurs steckenden Raspel-Räumahle 92 anzeigt (s. Fig. 1e und f).

Der Ausricht-Einsatz 3 ist an seinen Lateralseiten 33 keilartig abgeschrägt (Keilwinkel γ beträgt 4 bis 10 Grad, vorzugsweise 6 Grad), und zwar mindestens um den Schaftwinkel α. Damit ergibt sich einerseits eine hinreichend genaue Positionierung in der Linearführung 2, und andererseits kann - anders als bei einer echten Schwalbenschwanzführung - der Einsatz nach oben entnommen werden, und zwar am Ende des Ausrichtvorgangs (s. Fig. 14).

Es sei angemerkt, dass mit einem Räumfräser 37, welcher gegebenenfalls über einen Einstich 37' ähnlich dem Einstich 96 an der Raspel-Räumahle 92 verfügt und entsprechend über die Verengung 31' in die Öffnung 31 einsetzbar ist, eine Vorbearbeitung einer Höhlung in dem Markraum des Femurs vorgenommen werden kann (s. Fig. 16). Der Räumfräser 36 ist hierbei um denselben Schaftwinkel α verkippt wie die Raspel-Räumahle 92. So ist es ermöglicht, einen tiefliegenden Bereich für den Schaft der Prothese auszufräsen. Die maximale Frästiefe ist hierbei durch einen als Schaftbund ausgeführten Tiefenanschlag 37" begrenzt. Damit kann auch tief im Knochen die benötigte Aushöhlung hergestellt werden, und zwar im richtigen Winkel, ohne dass es dazu besonderer Fertigkeiten seitens des Operateurs bedarf.

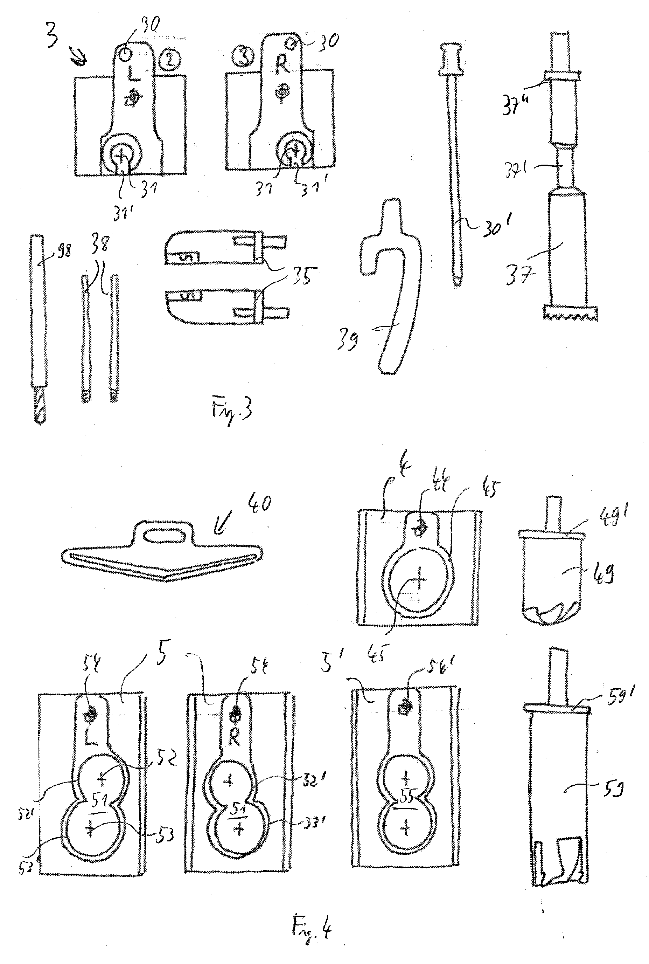

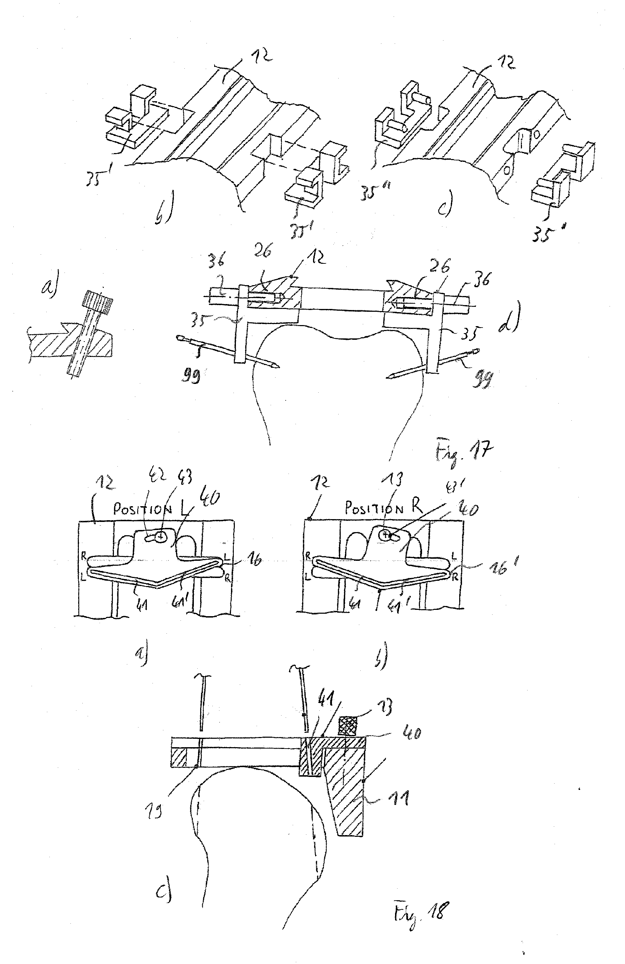

Im Regelfall wird der Basisrahmen 1 mit seiner Bezugsebene 10 direkt auf dem Ende des Femurs aufliegen. Jedoch ist dies nicht immer der Fall, sondern insbesondere in Fällen einer Reoperation und in anderen Fällen, bei denen bereits Knochenmaterial fehlt (wenn es bei einer vorhergehenden Operation abgetragen wurde oder aufgrund eines Defekts fehlt), können an der Unterseite 10 der Basisplatte 1 Abstandshalter 35 angeordnet sein (s. Fig. 3). Diese sind paarförmig ausgeführt und werden über Befestigungsstifte 36, welche in die Aufnahmebohrungen 26 gesteckt sind, an der Primärlehre 11 der Basisplatte 1 gehaltert (s. Fig. 15a). Sie sind in verschiedenen Dicken (s. Fig 15b) verfügbar, so dass hiermit eine Feineinstellung vorgenommen werden kann. Alternativen für den Abstandshalter sind in Fig. 17 dargestellt. Die einfachste besteht darin, dass am Rand des Basisrahmens Einstellschrauben angeordnet sind (s. Fig. 17a). Es können auch alternative Abstandshalter 35, 35' zum Unterbau vorgesehen sein, die an den Lateralseiten über Haken- oder Stiftverbindungen formschlüssig gehaltert sind (s. Fig. 17b und c). Diese können im unteren Bereich auch mit Stiftöffnungen zum Durchstecken von Befestigungsstiften 99 versehen sein (s. Fig. 17d).

Ist nun mittels des Ausricht-Einsatzes 3 die Position des Basisrahmens 1 definiert, so wird dieser durch Einbringen der Befestigungsstifte 99 in die Öffnungen 28 relativ zum Femur fixiert. Der Ausricht-Einsatz 3 und die Raspel-Räumahle 92 können nun abgenommen werden. Die zur Positionierung verwendeten Hilfsmittel, insbesondere die Ausrichtstangen 38 und der Taststift 30' werden ebenfalls entfernt.

Mittels einer an sich bekannten und nicht näher beschriebenen Knochensäge kann nun unter Verwendung der posterioren Sägeblattführung 19 eine Bearbeitung der Kondylen am dorsalen Ende vorgenommen werden (s. Fig. 18c). Im nächsten Schritt wird der Frontalsäge-Einsatz 40 in die entsprechende diagonale Wechselführung 16, 16' eingesetzt, und zwar abhängig davon, ob es sich um eine Implantation linksseitig oder rechtsseitig handelt. Die durch die doppelbogenförmige Ausnehmung 16, 16' gegebene Orientierung wird durch die bipolare Festlegung mittels des Langlochs 42, in dessen Endpositionen 43, 43' jeweils eine Befestigungsschraube 13 in die Zentralbefestigung 14 eingedreht ist, definiert. Es wird nun entlang der V-förmigen Sägeblattführungen 41, 41' die frontale Kondylenseite mittels der an sich bekannten Knochensäge bearbeitet (s. Fig. 18a, b).

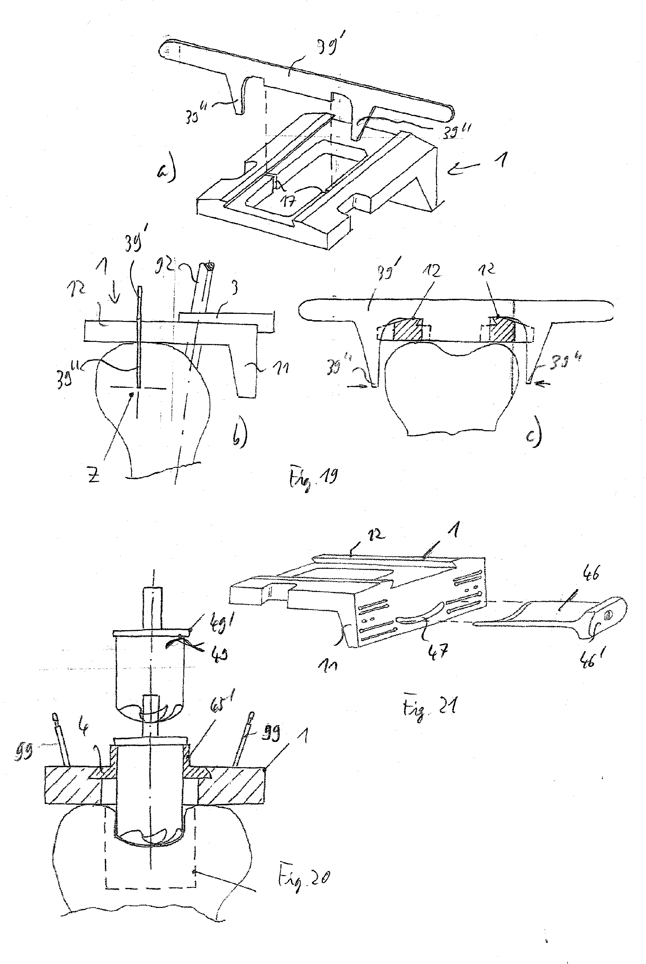

Im nächsten Schritt kann nun mittels der Fühlerlehre 39, die in den Schlitz 27 an dem Basisrahmen 1 eingesteckt ist, die Drehebene in einer Richtung und durch Einstecken in den Sägeschlitz 40, 40' die Drehebene in einer anderen, quer dazu orientierten Richtung festgelegt werden. Durch den Schnittpunkt der Drehebenen wird der Drehpunkt bestimmt. Ein alternatives Instrument ist in Fig. 19 dargestellt. Es umfasst einen Brückenträger als Richtlehre 39' mit beidseitig angeordneten Indexzungen 39". Der Brückenträger 39' wird in Aufnahmeschlitze 17 an dem Basisrahmen 1 eingesteckt, und durch die somit hergestellte formschlüssige Verbindung kann die Orientierung des Basisrahmens 1 eingestellt werden. Die Länge der Indexzungen 39" ist so bemessen, dass sie mit ihrem Ende die sich jeweils ergebende Lage des Drehpunkts Z anzeigen (s. Fig 19b und c). Die ermöglicht eine schnelle und gut kontrollierbare Ausrichtung.

Im folgenden Schritt wird der erste Fräs-Einsatz 4 in den Basisrahmen 1 eingesetzt und mittels der Zentralbefestigung 14, welche durch eine Öffnung 44 zugänglich ist, und der Befestigungsschraube 13 fixiert. Der Einsatz 4 weist eine große Zentralöffnung 45 mit einer nach oben abstehende Führhülse 45' auf, die eine Aufnahme für einen Räum-Fräser 49 bilden. Dieser weist in seinem oberen Bereich einen Bund 49' auf, welcher mit der Oberkante der Führhülse 45' derart zusammenwirkt, dass ein Tiefenanschlag für den Räumfräser 49 gebildet ist. Somit wird ein Teil der zur Implantation erforderlichen Höhlung im Markraum geschaffen, und zum anderen wird die von der Reibahle stehen gelassene Wandung im vorderen Bereich in ihrer Höhe definiert reduziert (s. Fig. 19). - Die Reduzierung der Höhe der Wandung im vorderen Bereich kann alternativ auch durch einen Meißel 46 erfolgen, wie in Fig. 20 dargestellt. Der Meißel 46 weist einen im Querschnitt kreisbogensegmentartigen Grundkörper mit einem auch als Tiefenanschlag fungierenden Schlagkopf 46' am hinteren Ende auf. In dem Basisrahmen 1 ist an der Primärlehre 11 ein zu der Querschnittsform des Meißels 46 komplementärer Führungsschlitz 47 vorgesehen.

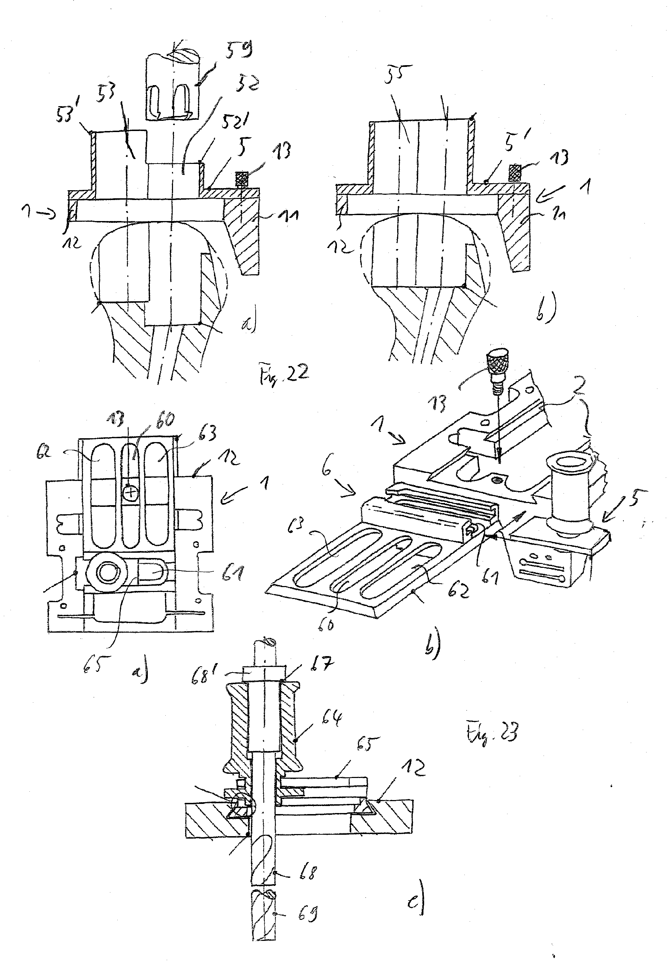

Im folgenden Schritt wird der erste Fräs-Einsatz 4 gegen einen zweiten Fräs-Einsatz 5 ausgetauscht. Dieser weist eine Doppelaufnahme 51 auf, welche im Querschnitt achtförmig ist und zwei Aufnahmepositionen 52, 53 für einen Volumenfräser 59 bildet. Die beiden Aufnahmen 52, 53 sind nicht mittig, sondern beide mit unterschiedlichem Versatz zur Seite (Lateralversatz) angeordnet. Jeder der beiden Aufnahmepositionen 52, 53 ist eine nach oben abstehende Hülse 52', 53' zugeordnet. Der Volumenfräser 59 weist ebenfalls in seinem oberen Bereich einen vorstehenden Bund 59' auf, welcher mit der Oberkante der der jeweiligen Aufnahme 52, 53 zugeordneten Hülse 52', 53' zusammenwirkt und damit einen Tiefenanschlag für den Volumenfräser 59 bildet (s. Fig. 22a). Damit kann ein Großteil des Hohlraums für die Aufnahme des Implantats vorgeformt werden. Es sei angemerkt, dass der zweite Fräs-Einsatz 5 genauso wie der erste Fräs-Einsatz 4 mittels einer mit der Zentralbefestigung fluchtenden Öffnung 54 eindeutig an dem Basisrahmen 1 positioniert ist.

Durch die verschiedenen Tiefenanschläge der beiden Aufnahmen 52, 53 der Doppelaufnahme 51 kann ein effizientes Ausräumen des Hohlraums im Knochen erreicht werden. Ist dies nicht erforderlich, so kann ein vereinfachter zweiter Fräs-Einsatz 5' vorgesehen sein, welcher eine Doppelaufnahme 55 ohne Lateralversatz aufweist. Dabei können dennoch die Tiefenanschläge auf gleicher Höhe liegen (s. Fig. 22b); es soll aber nicht ausgeschlossen sein, dass sie verschieden hoch sind (entsprechend der Darstellung in Fig. 22a).

Weiter sei angemerkt, dass der zweite Fräs-Einsatz 5 in zwei Version im Instrumentarium enthalten ist. Eine Version ist zur linksseitigen Implantation und eine zweite Version, welche spiegelsymmetrisch ist, zur rechtsseitigen Implantation ausgebildet (s. die mit "L" und "R" markierten zweiten Fräs-Einsätze 5 in Fig. 4).

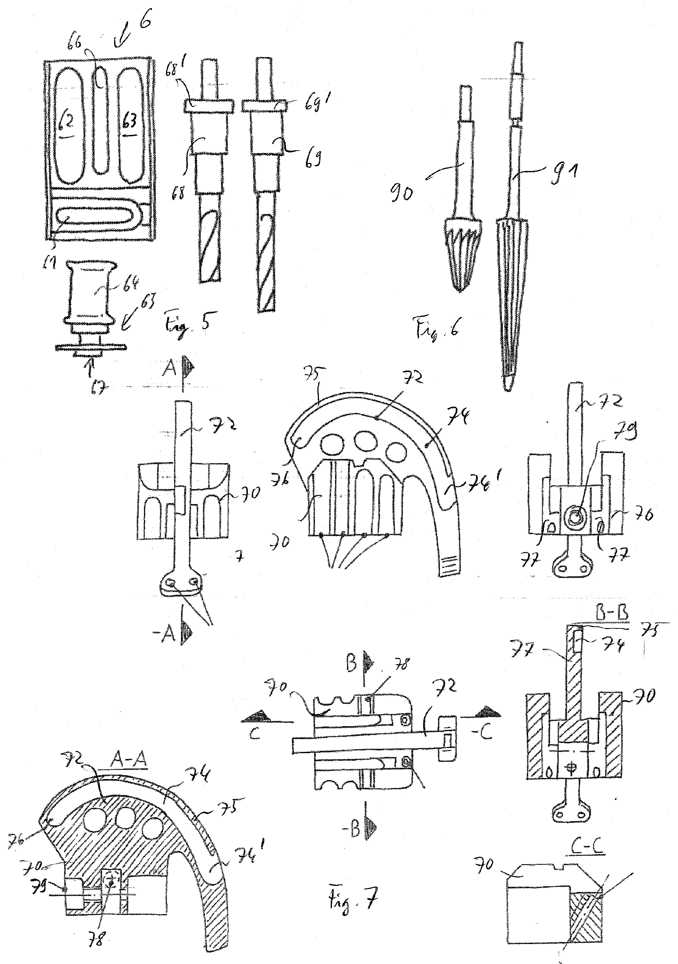

Im folgenden Schritt wird der zweite Fräs-Einsatz 5 ersetzt durch einen dritten Fräs-Einsatz 6, der als Kulissen-Einsatz ausgebildet ist (s. Fig. 5 und 23a-c). Dieser weist zwei T-förmig orientierte Kulissenfenster 60, 61 und zwei Beobachtungsfenster 62, 63 auf. Das Kulissenfenster 60 ist als Langloch ausgebildet und fungiert als Aufnahme für die Befestigungsschraube 13, mittels welcher der Kulissen-Einsatz an dem Basisrahmen 1 geführt ist. Damit kann der Kulissen-Einsatz 6 nach frontal und dorsal hin- und herbewegt werden. In das quer orientierte Kulissenfenster 61 ist ein Kulissengleiter 65 verschieblich eingesetzt, welcher einen Handgriff 64 aufweist mit einer Aufnahme 67 für ein Fräswerkzeug 68, 69. Die Fräswerkzeuge 68, 69 sind ein Vor- und ein End-Fräser, die mittels in unterschiedlichem Abstand zur Spitze angeordnetem Anschlagsbund 68', 69' für verschiedene Frästiefen ausgebildet sind (s. Fig. 5 und Fig. 23c). Sie können durch eine Zentralöffnung 67 im Handgriff 64 in den Kulissengleiter 63 durchgesteckt werden. Durch Bewegen des Kulissengleiters 63 entlang seines Kulissenfensters 61 und Bewegen des Kulissen-Einsatzes 6 entlang dem Kulissenfenster 60 (x/y-Bewegung) kann ein rechteckiger Hohlraumquerschnitt mit hoher Präzision ausgefräst werden. Dank der präzisen Kulissenführung kann der Hohlraum mit einer hohen Maßhaltigkeit erzeugt werden und so der kastenartige Aufnahmeraum für die Knieprothese im Femurhohlraum vorbereitet werden.

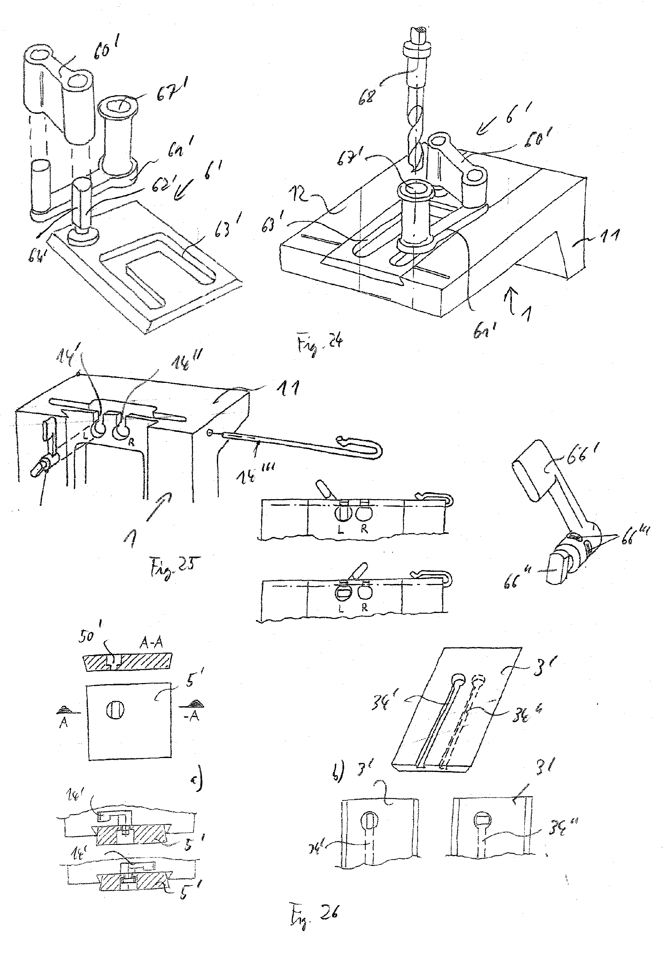

Eine alternative Ausführungsform für den Kulissen-Einsatz 6 ist in Fig. 24 dargestellt. Es handelt sich um einen Gelenkkulissen-Einsatz 6'. Er weist zwei gelenkig miteinander verbundene Führhebel 60', 61' auf. Mit einem Ende sind sie über einen Schwenkzapfen 62' drehbeweglich an einer Grundplatte des Einsatz 6' gelagert, und an dem anderen Ende ist eine Aufnahme 67' für die Fräswerkzeuge 68, 69 ausgebildet. An der Grundplatte der Kulissen-Einsatz 6' ist eine U-förmige Kulissenbahn 63' ausgeformt, in welcher das in die Aufnahme 67' gesteckte Fräswerkzeug 68, 69 durch die Führhebel 60', 61' zwangsgeführt ist. Der Schwenkzapfen 62' ist mit einer Abflachung 64' versehen, so dass die Führhebel 60', 61' nur in einer vorbestimmten Montageposition auf ihn aufgesetzt bzw. von ihm abgenommen werden können. Die Abflachung 64' ist dabei so ausgerichtet, dass in der Montageposition die Aufnahme 67' abseits der Kulissenbahn 63' steht. Damit ist sichergestellt, dass die Montage bzw. Demontage nur dann vorgenommen werden kann, wenn das Fräswerkzeug 68, 69 entfernt ist.

Eine zusätzliche Sicherung der Einsätze 3, 4 und 5 in dem Basisrahmen 1 ist in Fig. 25 dargestellt. Die Sicherungsöffnung 14 am Basisrahmen ist doppelt ausgeführt, und zwar eine linksseitige 14' und eine rechtsseitige 14". Die Bestigungsöffnungen 34, 44 und 54 an den Einsätzen 3, 4 und 5 sind als bis zum Rand hin laufende Nuten 34', 34" ausgeführt und lateral zueinander versetzt, je nachdem ob der jeweilige Einsatz zur links- oder rechtsseitigen Implantation vorgesehen ist. Ein Sicherungshebel 66' umfasst einen Knebel 66" am Ende eines Wellenstumpfs. Der Sicherungshebel 66' ist in der Fig. 25b dargestellten Orientierung von der Seite in eine der Befestigungsöffnungen 14', 14" einsetzbar. Durch Umlegen des Sicherungshebels 66' wird der Knebel 66" quergestellt, wodurch eine Verriegelung erreicht wird. Um ein versehentliches Betätigen des Sicherungshebels 66' zu vermeiden, ist vorzugsweise ein Sicherungsriegel 14''' vorgesehen, der von der Seite in den Basisrahmen 1 eingesteckt ist und an einer Abflachung 66''' des Wellenstumpfs bündig derart liegt, dass der Wellenstumpf und damit der Sicherungshebel 66' an einer Drehbewegung gehindert sind. Der Sicherungshebel 66' kann erst dann wieder verstellt werden, wenn der Sicherungsriegel 14''' entfernt ist. Zweckmäßigerweise sind die entsprechenden Einsätze an ihrer Unterseite mit einer hinterschnittenen Bohrung 50' im Falle eines gegen Heraushebens zu sichernden Einsatz 5' (s. Fig. 26a) und/oder mit der Nut 34', 34" und einem verbreitertem Ende im Falle eines gegen Verschiebens zu sichernden Einsatz 3' (s. Fig. 26b) versehen. Dargestellt sind in den beiden kleineren Abbildungen jeweils die geöffnete Stellung (oben in Fig. 26a bzw. links in Fig 26b) und die geschlossene Stellung (untern In Fig. 26a bzw. rechts in Fig. 26b).

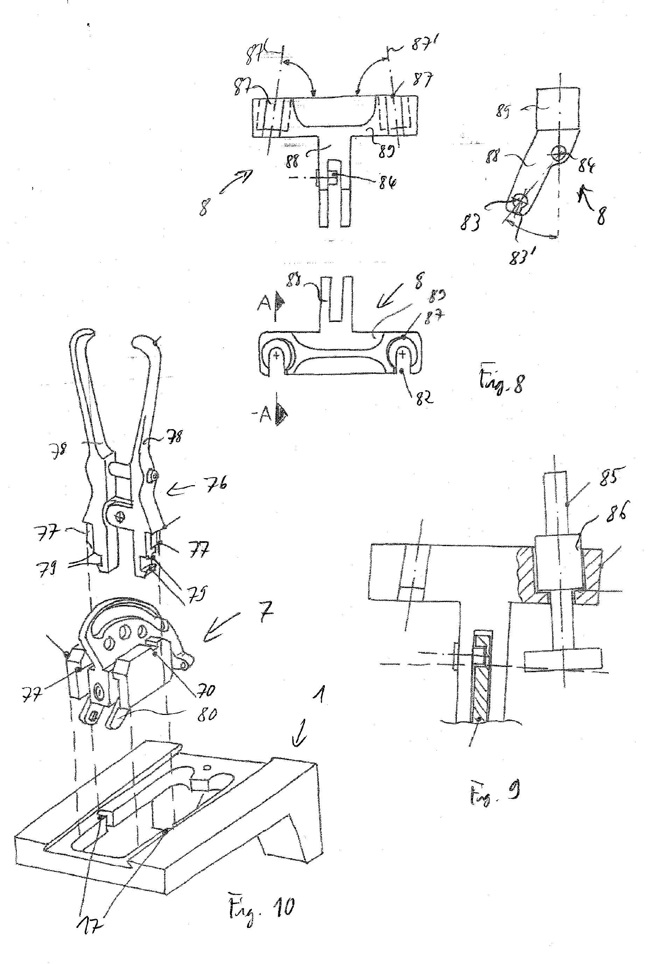

Nach der Ausarbeitung des kastenförmigen Aufnahmeraums im Femur folgt die Bearbeitung der Gleitbahnen an den Kondylen. Es wird insbesondere auf Fig. 7 bis 10 Bezug genommen. Zur Bearbeitung der Kondylen dient die Bogenfräslehre 7. Sie wird eingesetzt mittels einer Setzzange 76. Sie umfasst zwei Zangenhälften 78, die an ihren vorderen Enden zwei formschlüssig mit der Bogenfräslehre 7 zusammenwirkende Greifer 79 aufweist. An deren Außenseite sind voneinander wegweisende Ausricht-Nasen 77 ausgebildet. Sie sind so gestaltet, dass sie kongruent zur Form der Führungsschlitze 17 am Basisrahmen 1 sind. Damit ist durch Einsetzen der Setzzange 76 in den Basisrahmen 1, wobei die Ausricht-Nasen 77 formschlüssig in die Führungsschlitze 17 eingreifen, eine präzise Positionierung der Bogenfräslehre 7 relativ zum Basisrahmen 1 gewährleistet (s. Fig. 10). Es sei angemerkt, dass die Bogenfräslehre 7 in verschiedenen (vorzugsweise vier) Größen verfügbar ist, wobei die Positionierung mittels der Setzzange 76 durch formschlüssigen Eingriff in die Führungsschlitze 17 unabhängig von der jeweils verwendeten Größe in gleicher Weise erfolgt.

Die Bogenfräslehre 7 umfasst einen Grundkörper 70, dessen Grundgestalt etwa einem quaderartigen Kasten entspricht (s. Fig. 7). An seinen Lateralseiten sind vertikal von oben nach unten verlaufende Nuten 71 ausgebildet. Sie dienen zur festeren und positionswahrenden Verankerung in dem Hohlraum des Femurs. An der oberen Seite des Grundkörpers 70 ist ein nach oben abragendes Kreisbogensegment 72 einstückig ausgebildet, das einen Winkelbereich von etwa 100 bis 120 Grad überdeckt und im vorderen Bereich bis auf etwa die halbe Höhe des Grundkörpers 70 heruntergezogen ist. Im randnahen Bereich weist es an einer seiner Seitenflächen eine versenkte Führungsbahn 74 auf. Sie ist über einen Steg 75 zum oberen Rand des Kreisbogensegments 72 begrenzt, wobei in dem heruntergezogenen vorderen Bereich ein Fortsatz 74' der Führungsbahn 74 ausgebildet ist, der stegfrei ist. Damit ist eine Öffnung geschaffen, durch die ein Folger 84 des Führstücks 8 in die Führungsbahn 74 eingesetzt bzw. aus ihr entnommen werden kann. Dies kann nur in der Position erfolgen, wenn der Folger 84 sich im Bereich des Fortsatzes 74 befindet. Am anderen, hinteren Ende ist die Führungsbahn 74 geschlossen und bildet einen Anschlag 76 für den Folger 84.

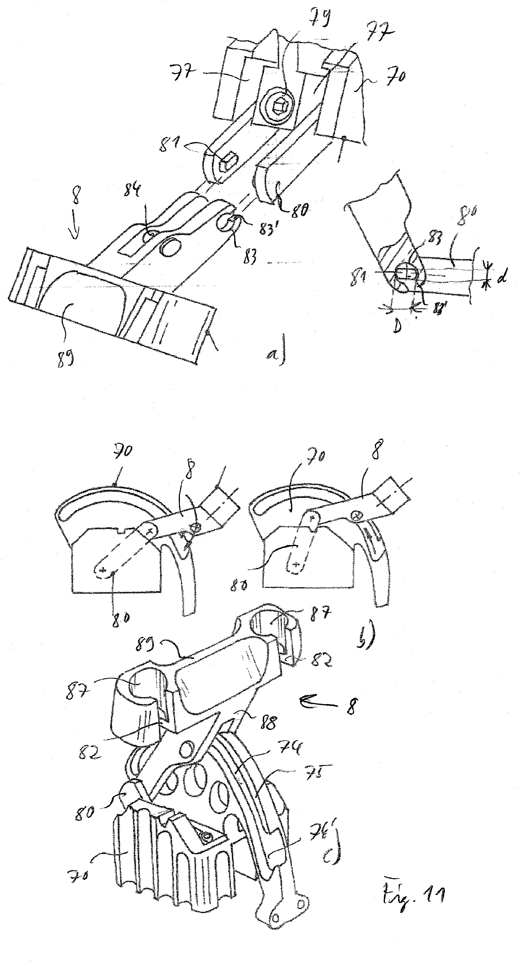

Von einer Rückseite des Grundkörpers 70 erstreckt sich ein Doppelschlitz 77 bis an die Oberseite des Grundkörpers 70. Quer dazu ist eine Aufnahmebohrung für eine Sicherungsschraube 79 angeordnet. Sie fungiert als Drehlager 78 für einen Kniehebel 80, dessen freies Ende aus dem Grundkörper 70 herausragt und der entlang des Doppelschlitzes 77 schwenkbar ist (s. Fig. 7). An seinem freien Ende trägt der Kniehebel 80 einen Drehzapfen 81, dessen Querschnitt etwa rechteckig mit geraden langen Seiten und kreisbogenförmig konvexen kurzen Seiten ist (s. Fig. 11a). Zwischen den kreisbogenförmig konvexen kurzen Seiten weist der Drehzapfen 81 einen Meridian größter Breite D und zwischen den geraden Seiten einen Meridian kleinster Breite d auf.

Das Führstück 8 ist generell von T-förmiger Gestalt mit einem Quersegment 89 und einem Längssegment 88 (s. Fig. 8). Am Quersegment 89 sind in den äußeren Bereichen jeweils eine Stufenbohrung 87 mit einer umlaufenden Schulter angeordnet. Sie sind mit ihrer Achse 87' so orientiert, dass sie einen Winkel α von 75 bis 85 Grad, vorzugsweise 80 Grad zum Quersegment 89 bilden. Damit divergieren die Achsen 87'. Die Stufenbohrung 87 bildet eine Führung für einen Kondylenfräser 85, der über eine seitliche Öffnung 82 in die Stufenbohrung 87 eingesetzt wird. Der Kondylenfräser 85 umfasst einen Fräskopf und einen Schaft, an dem eine vom Fräskopf beabstandete zylindrische Verdickung 86 ausgebildet ist. Indem sie auf der umlaufenden Schulter der Stufenbohrung 87 aufsetzt, begrenzt sie die Frästiefe.

Das Längssegment 88 des Führstücks 8 ist abgewinkelt; es bildet eine Winkel von etwa 15 bis 25 Grad, vorzugsweise 20 Grad zu der durch die Achsen 87' aufgespannten Ebene. Am Längssegment 88 ist der Folger 84 seitlich angeordnet, der das Führstück 8 längs der Führungsbahn 74 führt. Am freien Ende ist das Längssegment 88 gabelartig ausgeführt und mit einer Querbohrung versehen, die als Drehlagerhülse 83 fungiert. Sie ist über eine Verengung 83' zum freien Ende hin offen. Die Weite der Verengung 83' ist so bemessen, dass sie größer ist als der Meridian kleinster Breite 82 und kleiner als der Meridian größter Breite 82'. Sind die Drehlagerhülse 83 und der Drehzapfen 81 so orientiert, dass die Verengung 83' auf den Meridian kleinster Breite 82 trifft, kann das Führstück 8 mit seiner Drehlagerhülse 83 auf den Drehzapfen 81 aufgeschoben werden, und bei jeder anderen Orientierung ist der Drehzapfen 81 an einem Durchgang durch die Verengung 83' gehindert. Damit ergibt sich eine Winkelverriegelung, die nur in einer Stellung geöffnet bzw. geschlossen werden kann und im übrigen verriegelt ist (s. Fig. 11a und b).

Der Drehzapfen 81 ist so an dem Kniehebel 80 orientiert, dass ein Ankoppeln bzw. Abnehmen des Führstücks 8 nur in einer gestreckten Stellung erfolgen kann, wenn das Führstück 8 eine Linie mit dem Kniehebel 80 bildet (s. Fig 11a).

Ist das Ankoppeln erfolgt, wird das Führstück 8 mit seinem Folger 84 im Bereich des Fortsatz 74' in die Führungsbahn eingesetzt, wobei das Führstück 8 relativ zu dem Kniehebel 80 in abgewinkelter Stellung (also nicht mehr gestreckt) steht. Damit ist der Drehzapfen 81 an einem Durchgang durch die Verengung 83' gehindert. Das in die Führungsbahn 74 eingesetzte Führstück 8 ist damit sicher an dem Kniehebel 80 verriegelt (s. Fig. 11b).

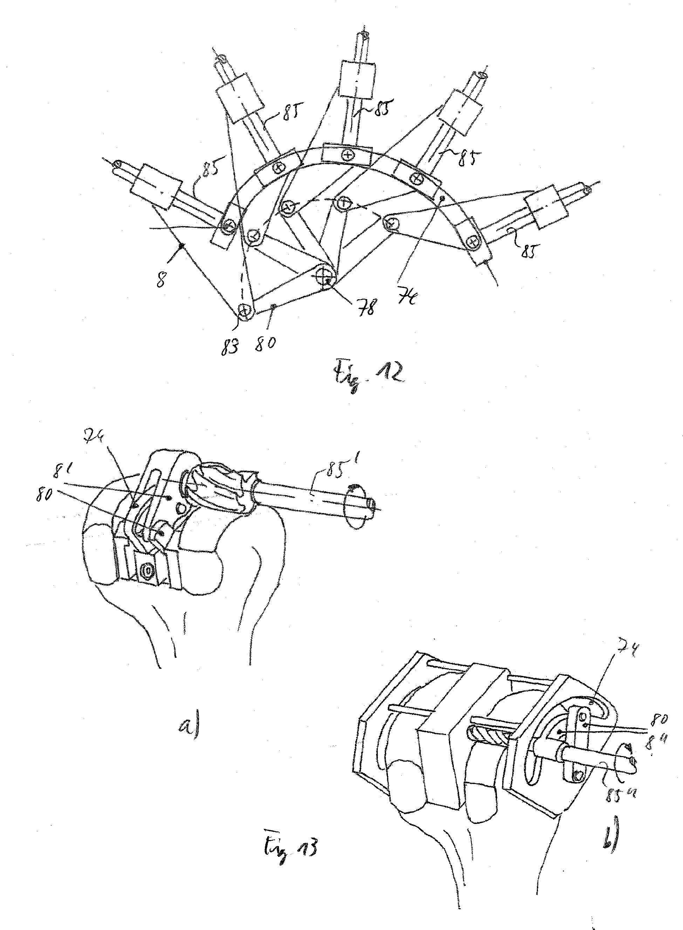

Die so erreichte Kinematik für die Führung des Kondylenfräsers 85 ist in Fig. 12 als Mehrphasendiagramm dargestellt. Unten in der Bildmitte ist das Drehlager 78 dargestellt. Es bildet einen festen Drehpunkt für die Kurvenführung bestehend aus Führungsbahn 74, Führstück 8, und Kniehebel 80 ist. Das Führstück 8 ist über seinen Folger 84 über den gesamten Schwenkbereich entlang der Führungsbahn 74 geführt. Die Führungsbahn 74 weist eine (in der Abbildung von links nach rechts) zunehmende Krümmung auf, wodurch sich der Radialabstand zwischen Drehlager 79 und Folger 84 vergrößert. In der Folge entfernt sich nicht nur der in das Führstück 8 eingesetzte Kondylenfräser 85 immer weiter von dem Drehlager 79 (er beschreibt also einen kontinuierlich größer werdenden Bogen), sondern es ändert sich auch seine Orientierung. Durch die Anlenkung über den Kniehebel 80 verdreht sich das Führstück, so dass die Achse 87 für den Fräser 85 zwar am Anfang der Schwenkbewegung auf das Drehlager 78 gerichtet ist, aber sich bei der Bewegung des Führstücks 8 entlang der Führungsbahn immer weiter nach dorsal (in Fig. 12 rechts) hin verdreht. Damit wird durch den Fräser 85 eine Kondylenform am Knochen erzeugt, deren Momentandrehpol bei der Flexion des Kniegelenks nicht stationär ist, sondern sich nach dorsal verschiebt. Damit ist für die mit dem erfindungsgemäßen Instrumentarium implantierte Knieprothese einen Bewegungsverlauf ermöglicht, der dem des natürlichen Knies praktisch voll entspricht und damit physiologisch äußerst günstig ist. Derartig implantierte Knieprothesen bieten beste Voraussetzungen für einen langfristigen Therapieerfolg, ohne dass es nach kurzer Zeit zu Revisionsoperationen kommen muss.

Eine alternative Ausführung des Führstücks für eine andere Kondylen-Fräsung ist in Fig. 13a, b dargestellt. Hierbei sind Führstücke 8', 8" vorgesehen, die einen Horizontal-Fräser 85', 85" aufnehmen. Bei der in Fig. 13a dargestellten Variante ist der Horizontal-Fräser 85' von der Seite in das Führstück 8' und dort einseitig gelagert. Die Bewegungskinematik entspricht der in Fig. 12 dargestellten. Die der in Fig. 13b dargestellten Variante sind der Kniehebel und das Führstück 8" außen angeordnet, und der Horizontal-Fräser 85" ist durch das Führstück 8" gesteckt. Bei dieser Variante ist es erforderlich, den Kniehebel und das Führstück 8" zur Bearbeitung der anderen Seite umzumontieren.

Das Instrumentarium umfasst weiter einen Ahlensatz zur Schaffung und Präparation einer Prothesenaufnahme im Femur. Der Ahlensatz (s. Fig. 6) umfasst eine Zugangsahle 90, die zum Öffnung der Markraumhöhle des Femurs dient. Mittels Reibahlen 91, die vorzugsweise in verschiedenen Längen und Durchmessern im Satz enthalten sind, wird die Markraumhöhle sukzessiv vergrößert. Weiter ist die spezielle Raspel-Räumahle 92 mit dem Anschlagteller 97 vorgesehen, die bereits vorstehen beschrieben wurden. Schließlich ist ein Stiftlochbohrer 98 mit Anschlag vorgesehen, der in die Bohrerführungen 18 eingesetzt wird zur Ausbildung von Aufnahmen für Verankerungszapfen (nicht dargestellt) an einem Kondylenteil der Kniegelenkendoprothese.

## Patentansprüche

1. Instrumentarium zum Einsetzen einer Gelenkprothese, insbesondere einer Femurkomponente einer Knieprothese, am Ende eines Knochens, insbesondere des Femurs, umfassend
a) einen Basisrahmen (1), der eine Führplatte (12) und eine davon seitlich abragende Primärlehre (11) aufweist,
b) eine Befestigungseinrichtung (99) zur positionsfesten Anordnung des Basisrahmens (1) an dem Knochen,
c) eine Bogenfräslehre (7) mit einem Grundkörper (70) und einem Führstück (8), wobei das Führstück (8) entlang einer gebogenen Führungsbahn (74) gegenüber dem Grundkörper (70) beweglich ist und es eine Aufnahme (87) für ein Abtragwerkzeug (85) aufweist, und
d) eine Ausrichteinrichtung (17, 77), welche die Bogenfräslehre (7) beim Einsetzen in den Basisrahmen (1) in eine eindeutig definierte Relativposition setzt.

2. Instrumentarium nach Anspruch 1, **dadurch gekennzeichnet, dass**
die gebogene Führungsbahn (74) eine nicht-konstante Krümmung aufweist, die vorzugsweise sich kontinuierlich längs der Führungsbahn (74) ändert, wobei vorzugsweise der jeweilige Krümmungsmittelpunkt (Z) sich um eine Strecke von 2 bis maximal 6 mm nach dorsal bewegt [falls die Krümmungsmittelpunkte stationär bleiben sollen, bitte korrigieren].

3. Instrumentarium nach Anspruch 1 oder 2, **dadurch gekennzeichnet, dass** das Führstück (8) über einen Folger (84) mit der Führungsbahn (74) und über ein Rotationslager (81, 83) schwenkbar an dem Grundkörper gelagert ist.

4. Instrumentarium nach Anspruch 3, **dadurch gekennzeichnet, dass**
das Rotationslager (81, 83) vom Folger (84) beabstandet angeordnet ist und einen beidseitig schwenkbar gelagerten Kniehebel (80) aufweist.

5. Instrumentarium nach Anspruch 3, **dadurch gekennzeichnet, dass** der Kniehebel (80) abnehmbar an dem Grundkörper (70) gehaltert ist.

6. Instrumentarium nach einem der Ansprüche 3 bis 5,
**dadurch gekennzeichnet, dass**
das Führstück (8) abnehmbar ausgeführt ist.

7. Instrumentarium nach Anspruch 6, **dadurch gekennzeichnet, dass**
eine Winkelverriegelung zwischen Führstück (8) und Grundkörper (70) vorgesehen ist, die durch Verbringen des Führstücks (8) in einen Fortsatz (74') der Führungsbahn (74) trennbar ist.

8. Instrumentarium nach Anspruch 7, **dadurch gekennzeichnet, dass**
die Winkelverriegelung eine Drehlagerhülse (83) und einen nicht kreisförmigen Drehzapfen (81) umfasst, die nur in einer Winkelposition des Führstücks (8) öffnet.

9. Instrumentarium nach Anspruch 8, **dadurch gekennzeichnet, dass**
die Drehlagerhülse (83) über eine Verengung (83') zur Seite offen ist, wobei der Drehzapfen (81) einen Meridian kleinster (d) und einen größter (D) Breite aufweist, und die Weite der Verengung (83') für den Durchgang des Meridians kleinster Breite (d), aber nicht größter Breite (D), ausreicht.

10. Instrumentarium nach einem der Ansprüche 3 bis 9, **dadurch gekennzeichnet, dass**
die Aufnahme (87) eine Achse (87') für das Abtragwerkzeug (85) definiert, die schiefwinklig zur Linie zwischen Folger (84) und Rotationslager (81, 83) ist, vorzugsweise im Bereich zwischen 10 und 35 Grad, weiter vorzugsweise zwischen 15 und 30 Grad.

11. Instrumentarium nach Anspruch 10. **dadurch gekennzeichnet, dass**
die Aufnahme (87) für das Abtragwerkzeug (85) an dem Führstück (8) mit einem Tiefenanschlag zusammenwirkt.

12. Instrumentarium nach Anspruch 11, **dadurch gekennzeichnet, dass**
der Tiefenanschlag als ein gestufter Sitz in der Aufnahme (87) ausgeführt ist, der vorzugsweise zu einer Seite hin offen ist.

13. Instrumentarium nach Anspruch 10, 11 oder 12, **dadurch gekennzeichnet, dass**
das Führstück (8) eine Doppelaufnahme für das Abtragwerkzeug (85) aufweist.

14. Instrumentarium nach Anspruch 13, **dadurch gekennzeichnet, dass**
die Doppelaufnahme divergierende Achsen (87') aufweist, so dass das Abtragwerkzeug (85) im eingesetzten Zustand nach außen weist.

15. Instrumentarium nach einem der Ansprüche 3 bis 9, **dadurch gekennzeichnet, dass**
das Führstück (8) mit dem Rotationslager (81, 83) und ggf. der Kniehebel (80) von einer Seite des Grundkörpers (70) auf die andere ummontierbar sind.

16. Instrumentarium nach einem der vorangehenden Ansprüche, **dadurch gekennzeichnet, dass** am Grundkörper (70) und/oder der Führungsbahn (74) Befestigungsbohrungen vorgesehen.

17. Instrumentarium nach einem der vorangehenden Ansprüche, **dadurch gekennzeichnet, dass** an der Führplatte (12) ein Ausricht-Einsatz (3) in definierter Position wechselbar angeordnet ist, der zur Aufnahme eines Ausrichtkörpers, vorzugsweise eines Knochenräumwerkzeugs (92), ausgebildet ist.

18. Instrumentarium nach Anspruch 17, **dadurch gekennzeichnet, dass** die Aufnahme (31) für den Ausrichtkörper einseitig offen ist.

19. Instrumentarium nach einem der vorangehenden Ansprüche, **dadurch gekennzeichnet, dass** es einen Frontalsäge-Einsatz (40) umfasst, der zwei V-förmig zueinander ausgerichtete Sägeschlitze (41) und eine bipolare Befestigung (42, 43) aufweist.

20. Instrumentarium nach einem der vorangehenden Ansprüche, **dadurch gekennzeichnet, dass** es Abstandshalter (35) in verschiedenen Höhen umfasst, die zu beidseitigen Anordnung am Rand der Führplatte (12) ausgebildet sind.

21. Instrumentarium nach einem der vorangehenden Ansprüche, **dadurch gekennzeichnet, dass** ein erster Fräs-Einsatz (4) wechselbar an der Führplatte (12) anbringbar ist, der eine definierte Aufnahme (45) für einen Räumfräser (49) bildet, die vorzugsweise den Fräser (49) in einer Position hält und einen Tiefenanschlag bildet.

22. Instrumentarium nach einem der vorangehenden Ansprüche, **dadurch gekennzeichnet, dass** es eine Fühlerlehre (39) umfasst, die in eine Steckaufnahme (27) an der Führplatte (12) winkelgenau einsteckbar ist.

23. Instrumentarium nach einem der vorangehenden Ansprüche, **dadurch gekennzeichnet, dass** ein zweiter Fräs-Einsatz (5) wechselbar an der Führplatte (12) anbringbar ist, der eine Doppelaufnahme (51) zur umsteckbaren Aufnahme eines Volumenfräsers (59) bildet, wobei die Doppelaufnahme (51) vorzugsweise verschiedene Tiefenanschläge und Lateralversatz aufweist.

24. Instrumentarium nach einem der vorangehenden Ansprüche, **dadurch gekennzeichnet, dass** ein zweiter Hilfsfräs-Einsatz (5') wechselbar an der Führplatte (12) anbringbar ist, der eine Doppelaufnahme (55) zur umsteckbaren Aufnahme eines Volumenfräsers (59) bildet, wobei die Doppelaufnahme (55) vorzugsweise identische Tiefenanschläge und/oder keinen Lateralversatz aufweist.

25. Instrumentarium nach einem der vorangehenden Ansprüche, **dadurch gekennzeichnet, dass** ein dritter Fräs-Einsatz (6) an der Führplatte anbringbar ist, der eine Kulissenführung (61) für einen Tiefenfräser (68, 69) bildet, wobei der Tiefenfräser (68, 69) vorzugsweise in einen Kulissengleiter (65) aufgenommen ist.

26. Instrumentarium nach Anspruch 25, **dadurch gekennzeichnet, dass** der Kulissengleiter (65) einen Handgriff (64) aufweist.

27. Instrumentarium nach Anspruch 26, **dadurch gekennzeichnet, dass** ein Tiefenanschlag (67) vorgesehen ist, der vorzugsweise so mit dem Tiefenfräser (68) und einem zweiten Tiefenfräser (69) zusammenwirkt, dass verschiedene Frästiefen erreicht werden.

28. Instrumentarium nach einem der Ansprüche 25 bis 27, **dadurch gekennzeichnet, dass** ein Kulissenfenster (61) vorgesehen sind, in das der Kulissengleiter (65) aufnehmbar ist.

29. Instrumentarium nach einem der Ansprüche 25 bis 28, **dadurch gekennzeichnet, dass** die Kulissenführung zwei gelenkig miteinander verbundene Führhebel (60', 61') umfasst, die an einem Ende die Aufnahme (67') für den Tiefenfräser (68) angeordnet und am anderen Ende schwenkbar an einem Einsatz (6') an der Führplatte (12) angeordnet ist.

30. Instrumentarium nach einem der vorangehenden Ansprüche, **dadurch gekennzeichnet, dass** die Führplatte (12) eine Linearführung (2), vorzugsweise eine Schwalbenschwanzführung, für verschiedene Einsätze (3, 4, 5) aufweist.

31. Instrumentarium nach einem der vorangehenden Ansprüche, **dadurch gekennzeichnet, dass** es Bogenfräslehren (7) in verschiedenen Größen umfasst.

32. Instrumentarium nach einem der vorangehenden Ansprüche, **dadurch gekennzeichnet, dass** eine Setzzange (76) für die Bogenfräslehre (7) vorgesehen ist, welche sie formschlüssig in definierter Position ergreift und über Ausrichtnasen (77) mit der Ausrichteinrichtung (17) so zusammenwirkt, dass die Bogenfräslehre (7) in eindeutiger Relativposition zur Führplatte (12) eingesetzt wird.

33. Instrumentarium nach einem der vorangehenden Ansprüche, **dadurch gekennzeichnet, dass** es seitlich am Basisrahmen (1) anzuordnende, voneinander weg weisende Ausrichtstangen (38) umfasst.

34. Instrumentarium nach einem der Ansprüche 1 bis 33, **dadurch gekennzeichnet, dass** es einen Bohrer (98) mit Tiefenanschlag zur Schaffung von Aufnahmen von Verankerungszapfen der Gelenkprothese umfasst.

35. Instrumentarium nach einem der vorangehenden Ansprüche, **dadurch gekennzeichnet, dass** es verschiedene Ahlen (90, 91) zum Ausräumen einer Höhlung am Knochen, insbesondere eines Markraums am Femur, umfasst.

36. Instrumentarium nach einem der vorangehenden Ansprüche, **dadurch gekennzeichnet, dass** es eine Raspel-Räumahle (92) umfasst, deren Schaft einen Einstich (96) mit einer verringerten Dicke aufweist, der zur Aufnahme eines Anschlagtellers (97) ausgebildet ist.

37. Instrumentarium nach Anspruch 36, **dadurch gekennzeichnet, dass** die Raspel-Räumahle (92) zwei, drei oder vier Schneidkanten (93) aufweist.

38. Instrumentarium nach Anspruch 37, **dadurch gekennzeichnet, dass** Zähne (94) an den Schneidkanten (93) mit relativem Höhenversatz angeordnet sind.

39. Instrumentarium nach einem der vorangehenden Ansprüche, **dadurch gekennzeichnet, dass** eine Richtlehre (39') zur Positionierung des Basisrahmens (1) vorgesehen ist, die an der Ausrichteinrichtung (17) angreift.
